# EUROPEAN PATENT APPLICATION

(11) **EP 4 579 673 A1**
(43) Date of publication of application: **02.07.2025**
(21) Application number: 24217190.8
(22) Date of filing: 03.12.2024
(51) Int. Cl.: G16H 15/00, A61B 5/00, G16H 40/63, G16H 40/67, G16H 50/20, G16H 50/30, G16H 50/70

(54) **INTELLIGENT CLINICAL USER INTERFACES**

(30) Priority: 28.12.2023 US 202318399032
(71) Applicant: GE Precision Healthcare LLC, Waukesha, WI 53188 (US)
(72) Inventor: AHMED, Zuber, 560066 Bangalore (IN)
(74) Representative: Fennell, Gareth Charles

(57) **Abstract**

Systems or techniques that facilitate intelligent clinical user interfaces are provided. In various embodiments, a system can access attribute data (e.g., 104) corresponding to a medical patient and can access real-time measurements (e.g., 108) of a plurality of vital sign categories (e.g., 106) of the medical patient. In various aspects, the system can identify, via execution of a machine learning model (e.g., 302) on the attribute data, which of the plurality of vital sign categories is clinically relevant to the medical patient, thereby yielding an identified vital sign category (e.g., 402). In various instances, the system can visually render, on a graphical user interface (GUI) (e.g., 502), real-time measurements (e.g., 602) of the identified vital sign category. In various cases, the system can determine, via the machine learning model, a visual style (e.g., 404) to aid the medical patient's viewing of the GUI, and the system can visually render the real-time measurements of the identified vital sign category according to the visual style.

## Description

### TECHNICAL FIELD

The subject disclosure relates generally to clinical user interfaces, and more specifically to intelligent clinical user interfaces.

### BACKGROUND

When given a medical patient whose vital signs are being monitored, it can be desired to display the medical patient's vital sign measurements on a graphical user interface. Existing techniques do so in static, standardized fashion.

### SUMMARY

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate intelligent clinical user interfaces are described.

According to one or more embodiments, a system is provided. The system can comprise a non-transitory computer-readable memory that can store computer-executable components. The system can further comprise a processor that can be operably coupled to the non-transitory computer-readable memory and that can execute the computer-executable components stored in the non-transitory computer-readable memory. In various embodiments, the computer-executable components can comprise an access component that can access attribute data corresponding to a medical patient and that can access real-time measurements of a plurality of vital sign categories of the medical patient. In various aspects, the computer-executable components can comprise a model component that can identify, via execution of a machine learning model on the attribute data, which of the plurality of vital sign categories is clinically relevant to the medical patient, thereby yielding an identified vital sign category. In various instances, the computer-executable components can comprise a display component that can visually render, on a graphical user interface, real-time measurements of the identified vital sign category. In various cases, the model component can determine, via the execution of the machine learning model, a visual style that is predicted to aid the medical patient's viewing or an attending clinician's viewing of the graphical user interface, and the display component can visually render the real-time measurements of the identified vital sign category according to the visual style.

According to one or more embodiments, a computer-implemented method is provided. In various embodiments, the computer-implemented method can comprise accessing, by a device operatively coupled to a processor, attribute data corresponding to a medical patient and real-time measurements of a plurality of vital sign categories of the medical patient. In various aspects, the computer-implemented method can comprise identifying, by the device and via execution of a machine learning model on the attribute data, which of the plurality of vital sign categories is clinically relevant to the medical patient, thereby yielding an identified vital sign category. In various instances, the computer-implemented method can comprise visually rendering, by the device and on a graphical user interface, real-time measurements of the identified vital sign category. In various cases, the computer-implemented method can comprise determining, by the device and via the execution of the machine learning model, a visual style that is predicted to aid the medical patient's viewing or an attending clinician's viewing of the graphical user interface, wherein the real-time measurements of the identified vital sign category can be visually rendered according to the visual style.

According to one or more embodiments, a computer program product for facilitating intelligent clinical user interfaces is provided. In various embodiments, the computer program product can comprise a non-transitory computer-readable memory having program instructions embodied therewith. In various aspects, the program instructions can be executable by a processor to cause the processor to access attribute data corresponding to a medical patient and real-time measurements of a plurality of vital sign categories of the medical patient. In various instances, the program instructions can be further executable to cause the processor to identify, via execution of a machine learning model on the attribute data, which of the plurality of vital sign categories is clinically relevant to the medical patient, thereby yielding an identified vital sign category. In various cases, the program instructions can be further executable to cause the processor to convey, via an electronic user interface, real-time measurements of the identified vital sign category.

### DESCRIPTION OF THE DRAWINGS

FIG. 1 illustrates a block diagram of an example, non-limiting system that facilitates intelligent clinical user interfaces in accordance with one or more embodiments described herein.
FIG. 2 illustrates an example, non-limiting block diagram showing a plurality of patient vital sign categories and a plurality of real-time vital sign measurements in accordance with one or more embodiments described herein.
FIG. 3 illustrates a block diagram of an example, non-limiting system including a deep learning neural network and one or more GUI-related inferences that facilitates intelligent clinical user interfaces in accordance with one or more embodiments described herein.
FIG. 4 illustrates an example, non-limiting block diagram showing how a deep learning neural network can generate one or more GUI-related inferences in accordance with one or more embodiments described herein.
FIG. 5 illustrates a block diagram of an example, non-limiting system including a patient-tailored GUI that facilitates intelligent clinical user interfaces in accordance with one or more embodiments described herein.
FIG. 6 illustrates an example, non-limiting block diagram showing a patient-tailored GUI in accordance with one or more embodiments described herein.
FIG. 7 illustrates a block diagram of an example, non-limiting system including clinician metadata that facilitates intelligent clinical user interfaces in accordance with one or more embodiments described herein.
FIG. 8 illustrates an example, non-limiting block diagram showing how a deep learning neural network can take clinician metadata into account in accordance with one or more embodiments described herein.
FIG. 9 illustrates a block diagram of an example, non-limiting system including a training component and a training dataset that facilitates intelligent clinical user interfaces in accordance with one or more embodiments described herein.
FIG. 10 illustrates an example, non-limiting block diagram of a training dataset in accordance with one or more embodiments described herein.
FIG. 11 illustrates an example, non-limiting block diagram showing how a deep learning neural network can be trained in accordance with one or more embodiments described herein.
FIG. 12 illustrates a flow diagram of an example, non-limiting computer-implemented method that facilitates intelligent clinical user interfaces in accordance with one or more embodiments described herein.
FIG. 13 illustrates a block diagram of an example, non-limiting operating environment in which one or more embodiments described herein can be facilitated.
FIG. 14 illustrates an example networking environment operable to execute various implementations described herein.

### DETAILED DESCRIPTION

The following detailed description is merely illustrative and is not intended to limit embodiments or application/uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

A medical patient's vital signs (e.g., body temperature, blood pressure, heart rate) can be measured in real-time via any suitable medical equipment (e.g., thermometers, blood pressure gauges, pulse monitors). During such real-time monitoring, it can be desired to display the medical patient's vital sign measurements on a graphical user interface (GLTI). For example, such GUI can be rendered on an electronic screen of a smart device or of a hospital computing console. Accordingly, the medical patient (or their attending clinician) can view the GUI, so as to become apprised of the current health status of the medical patient.

Existing techniques facilitate such display in static, standardized fashion. In particular, when existing techniques are implemented, a clinical GUI displays a uniform set of vital sign measurements in a uniform way, no matter the medical patient to whom those vital sign measurements correspond. In other words, the inventor of various embodiments described herein recognized that the GUIs of such existing techniques are crafted in cookie-cutter fashion and are not customized or tailored to individual medical patients.

Accordingly, systems or techniques that can address one or more of these technical problems can be desirable.

Various embodiments described herein can address one or more of these technical problems. One or more embodiments described herein can include systems, computer-implemented methods, apparatus, or computer program products that can facilitate intelligent clinical user interfaces. In particular, when given a medical patient whose vital signs are being monitored in real-time, various embodiments described herein can involve generating a clinical GUI that is tailored to that given medical patient, by leveraging a machine learning model. Indeed, in some cases, the machine learning model can be configured to identify which specific vital sign is most clinically relevant to the given medical patient, and the tailored clinical GUI can be rendered so as to prominently depict the real-time measurements of that identified vital sign (e.g., the real-time measurements of other, non-relevant vital signs can be minimized in or omitted from the tailored clinical GUI). In other cases, the machine learning model can be further configured to determine a visual style (e.g., font sizes, color schemes) that would suit or be comfortable for the given medical patient, and the tailored clinical GUI can be rendered in compliance with that visual style. In yet other cases, the machine learning model can be further configured to determine a threshold value that delineates an upper or lower bound of healthy or acceptable behavior of the identified vital sign of the given medical patient, and the tailored clinical GUI can depict an alert whenever the real-time measurements of the given medical patient fail to satisfy that threshold value. In other words, the clinical GUIs of existing techniques, as recognized by the present inventor, can be considered as one-size-fits-all interfaces that fail to take into consideration clinical idiosyncrasies of different medical patients. In contrast, various embodiments described herein can leverage artificial intelligence to generate clinical GUIs that are uniquely tailored or adjusted to the clinical idiosyncrasies of different medical patients (e.g., different vital signs might be more or less clinically relevant to different medical patients; different visual styles might be more or less suited to or comfortable for different medical patients; the upper or lower bounds of healthy vital sign behavior might be different for different medical patients). Therefore, various embodiments described herein can be considered as being advantageous over existing techniques.

Various embodiments described herein can be considered as a computerized tool (e.g., any suitable combination of computer-executable hardware or computer-executable software) that can facilitate intelligent clinical user interfaces. In various aspects, such computerized tool can comprise an access component, a model component, or a display component.

In various embodiments, there can be patient metadata. In various aspects, the patient metadata can correspond to any suitable medical patient. In various instances, the patient metadata can indicate, quantify, convey, or otherwise represent any suitable physical characteristics, attributes, or demographics of the medical patient (e.g., age, sex, weight, height, health habits, pathologies, prostheses, symptoms, occupation, education level). In some cases, the patient metadata can represent or otherwise be derived from all or a portion of a medical history of the medical patient.

In various embodiments, there can be a plurality of vital sign categories (e.g., blood pressure category, heart rate category, body temperature category). In various aspects, a plurality of real-time vital sign measurements respectively corresponding to the plurality of vital sign categories can be tracked or recorded for the medical patient (e.g., real-time blood pressure measurements of the medical patient can be tracked; real-time heart rate measurements of the medical patient can be tracked; real-time body temperature measurements of the medical patient can be tracked). In various instances, the plurality of real-time vital sign measurements can be recorded or otherwise captured by any suitable medical diagnostic equipment (e.g., blood pressure gauge, pulse monitor, thermometer).

In various cases, it can be desired to display various of the real-time vital sign measurements on a clinical GUI that is viewable by or to the medical patient. As described herein, the computerized tool can facilitate such display.

In various embodiments, the access component of the computerized tool can electronically receive or otherwise electronically access the patient metadata or the plurality of real-time vital sign measurements. In some aspects, the access component can electronically retrieve the patient metadata or the plurality of real-time vital sign measurements from any suitable centralized or decentralized data structures (e.g., graph data structures, relational data structures, hybrid data structures), whether remote from or local to the access component. In other aspects, the access component can electronically retrieve the patient metadata or the plurality of real-time vital sign measurements from whatever medical equipment they are measured, captured, or stored by. In any case, the access component can electronically obtain or access the patient metadata or the plurality of real-time vital sign measurements, such that the access component can serve as a conduit by which or through which other components of the computerized tool can electronically interact with (e.g., read, write, edit, copy, manipulate) the patient metadata or with the plurality of real-time vital sign measurements.

In various embodiments, the model component of the computerized tool can electronically store, maintain, control, or otherwise access a deep learning neural network. In various aspects, the deep learning neural network can exhibit any suitable deep learning internal architecture. For example, the deep learning neural network can include any suitable numbers of any suitable types of layers (e.g., input layer, one or more hidden layers, output layer, any of which can be convolutional layers, dense layers, non-linearity layers, long short-term memory (LSTM) layers, pooling layers, batch normalization layers, or padding layers). As another example, the deep learning neural network can include any suitable numbers of neurons in various layers (e.g., different layers can have the same or different numbers of neurons as each other). As yet another example, the deep learning neural network can include any suitable activation functions (e.g., softmax, sigmoid, hyperbolic tangent, rectified linear unit) in various neurons (e.g., different neurons can have the same or different activation functions as each other). As still another example, the deep learning neural network can include any suitable interneuron connections or interlayer connections (e.g., forward connections, skip connections, recurrent connections).

In various instances, the deep learning neural network can be configured to receive as input medical information and to produce as output various GUI-related predictions based on that inputted medical information. Accordingly, the model component can, in various cases, execute the deep learning neural network on the patient metadata, and such execution can cause the deep learning neural network to produce one or more GUI-related inferences that pertain to the medical patient. More specifically, the model component can feed the patient metadata to an input layer of the deep learning neural network. In various aspects, the patient metadata can complete a forward pass through one or more hidden layers of the deep learning neural network. In various instances, an output layer of the deep learning neural network can compute the one or more GUI-related inferences, based on activation maps provided by the one or more hidden layers.

In various aspects, the one or more GUI-related inferences can be any suitable electronic data that represents, conveys, indicates, describes, or otherwise characterizes substantive or aesthetic content that would be appropriate (in the opinion of the deep learning neural network) to be viewed by the medical patient.

As a non-limiting example, the one or more GUI-related inferences can comprise a relevant vital sign category indicator. In various aspects, the relevant vital sign category indicator can be any suitable electronic data that indicates or specifies which one (or more) of the plurality of vital sign categories is most clinically relevant (as determined by the deep learning neural network) to the medical patient. Indeed, different medical patients can have different clinical idiosyncrasies (e.g., different physical attributes, different medications, different pathologies, or different severities of pathologies). Moreover, different vital sign categories can be considered as being more relevant or less relevant to different clinical idiosyncrasies. Thus, certain vital sign categories can be considered as being relevant to some medical patients, and those certain vital sign categories can instead be considered as being irrelevant to other medical patients. Accordingly, in various instances, the patient metadata can be considered as representing the particular clinical idiosyncrasies of the medical patient, and the relevant vital sign category indicator can represent or specify which of the plurality of vital sign categories the deep learning neural network has inferred or predicted is or are relevant to those particular clinical idiosyncrasies.

For instance, suppose that the patient metadata indicates that the medical patient has a known history of suffering from glaucoma. In such case, the deep learning neural network can infer that blood pressure is a relevant vital sign category with respect to glaucoma (e.g., since glaucoma is significantly intertwined with excessive intraocular pressure). Accordingly, the relevant vital sign category indicator can indicate or specify that blood pressure is particularly relevant to the medical patient and that other vital sign categories (e.g., heart rate, body temperature) are not as relevant to the medical patient. As another instance, suppose that the patient metadata indicates that the medical patient is on an antimicrobial medication regimen. In such case, the deep learning neural network can infer that body temperature is a relevant vital sign category with respect to antimicrobial medication (e.g., since antimicrobial medications are often associated with inducing fevers). Accordingly, the relevant vital sign category indicator can indicate or specify that body temperature is particularly relevant to the medical patient and that other vital sign categories are not as relevant to the medical patient.

As another non-limiting example, the one or more GUI-related inferences can comprise a recommended visual style indicator. In various aspects, the recommended visual style indicator can be any suitable electronic data that indicates or specifies various visually stylistic elements (e.g., font size, color scheme, brightness, contrast, geometric pattern) that would assist, ease, or otherwise improve (in the opinion of the deep learning neural network) GUI viewability for the medical patient. Indeed, as mentioned above, different medical patients can have different clinical idiosyncrasies. Furthermore, different visually stylistic elements can be considered as being more comfortable to view or less comfortable to view depending upon such different clinical idiosyncrasies. Thus, certain visually stylistic elements can be considered as being comfortably viewed by some medical patients, and those certain visually stylistic elements can instead be considered as being uncomfortably viewed by other medical patients. Accordingly, in various instances, the patient metadata can be considered as representing the particular clinical idiosyncrasies of the medical patient, and the recommended visual style indicator can represent or specify various visually stylistic elements that the deep learning neural network has inferred or predicted are comfortable or well-suited for those particular clinical idiosyncrasies.

For instance, suppose that the patient metadata indicates that the medical patient is elderly. In such case, the deep learning neural network can infer that the elderly often have an easier time viewing large font sizes and a harder time viewing small font sizes (e.g., since visual acuity generally declines with age). Accordingly, the recommended visual style indicator can indicate or specify that a large font size would be appropriate for the medical patient or that a small font size would be inappropriate for the medical patient. As another instance, suppose that the patient metadata indicates that the medical patient is a young child. In such case, the deep learning neural network can infer that children have an easier time viewing colorful GUIs and a harder time viewing monotonously-colored GUIs (e.g., since color variety can help to maintain a child's attention). Accordingly, the recommended visual style indicator can indicate or specify that a rainbow color scheme would be appropriate for the medical patient or that a monochromatic color scheme would be inappropriate for the medical patient.

As even another non-limiting example, the one or more GUI-related inferences can comprise a recommended vital sign threshold. In various aspects, the recommended vital sign threshold can be any suitable scalar that demarcates, delineates, or otherwise serves as a bound for healthy behavior of whichever vital sign category is specified by the relevant vital sign category indicator. Indeed, as mentioned above, different medical patients can have different clinical idiosyncrasies. Further still, what vital sign behavior qualifies as healthy or acceptable can differ across such different clinical idiosyncrasies. Thus, a certain vital sign behavior can be considered as being healthy or acceptable for some medical patients, and that certain vital sign behavior can instead be considered as being unhealthy or unacceptable for other medical patients. Accordingly, in various instances, the patient metadata can be considered as representing the particular clinical idiosyncrasies of the medical patient, the relevant vital sign category indicator can specify a vital sign category that the deep learning neural network has inferred or predicted is relevant to those particular clinical idiosyncrasies, and the recommended vital sign threshold can be a numerical marker that the deep learning neural network has inferred or predicted represents a bound of healthy or acceptable behavior of that vital sign category for those particular clinical idiosyncrasies.

For instance, suppose that the relevant vital sign category indicator specifies blood pressure, and further suppose that the patient metadata indicates that the medical patient has a known history of suffering from glaucoma. Now, the deep learning neural network can infer that glaucoma is associated with high blood pressure, and so the deep learning neural network can generate the recommended vital sign threshold as an upper bound blood pressure value. If the patient metadata indicates that the medical patient has experienced only mild glaucoma, the deep learning neural network can cause that upper bound blood pressure value to be a less conservative value (e.g., the deep learning neural network can infer that 135 millimeters of mercury (mmHG) can be the upper bound of acceptable blood pressure for the medical patient). Instead, if the patient metadata indicates that the medical patient has experienced severe glaucoma, the deep learning neural network can cause that upper bound blood pressure value to be a more conservative value (e.g., the deep learning neural network can infer that 120 mmHG can be the upper bound of acceptable blood pressure for the medical patient).

In any case, the model component can execute the deep learning neural network on the patient metadata, and such execution can yield the one or more GUI-related inferences.

In various embodiments, the display component of the computerized tool can electronically control any suitable electronic display (e.g., computer screen, smart phone screen). In various aspects, the display component can visually render, on that electronic display, a patient-tailored GUI, based on the one or more GUI-related inferences produced by the deep learning neural network.

In particular, as mentioned above, the medical patient can be associated with the plurality of vital sign categories, and real-time measurements respectively corresponding to the plurality of vital sign categories can be captured or recorded from the medical patient by any suitable medical equipment. In various aspects, the patient-tailored GUI can prominently depict, illustrate, or otherwise show in textual, numerical, or graphical fashion the real-time measurements that correspond to whichever vital sign category is specified by the relevant vital sign category indicator. Indeed, in some instances, the patient-tailored GUI can depict the real-time measurements corresponding to the relevant vital sign category indicator and can exclude, omit, hide, or otherwise not depict any real-time measurements that correspond to the remainder of the plurality of vital sign categories. In other instances, however, the patient-tailored GUI can depict the real-time measurements corresponding to the relevant vital sign category indicator and can depict the real-time measurements corresponding to the remainder of the plurality of vital sign categories, but the latter can be illustrated in a smaller size (e.g., one or more multiples smaller) than the former (e.g., the latter can be visually minimized such that visual attention is drawn to the former). In any case, the patient-tailored GUI can be considered as prominently visually displaying the real-time measurements of whatever vital sign category that the deep learning neural network has inferred or predicted is clinically relevant to the medical patient, hence the term "patient-tailored."

Furthermore, in various aspects, the display component can render the patient-tailored GUI in compliance with the recommended visual style indicator. In other words, the recommended visual style indicator can specify various visually stylistic elements (e.g., font size, color scheme, brightness) that the deep learning neural network has inferred or predicted would be easy, appropriate, or comfortable for the medical patient to view, and the patient-tailored GUI can be constructed of such visually stylistic elements, again hence the term "patient-tailored."

Further still, in various instances, the display component can continuously, continually, or periodically check whether the real-time measurements that are prominently depicted by the patient-tailored GUI satisfy the recommended vital sign threshold. If the display component determines that those real-time measurements fail to satisfy the recommended vital sign threshold (e.g., if those measurements exceed an upper bound represented by the recommended vital sign threshold; if those measurements fall beneath a lower bound represented by the recommended vital sign threshold), then the display component can cause the patient-tailored GUI to depict or illustrate an electronic alert indicating such failure. In other words, the patient-tailored GUI can depict a visible alert or warning notification whenever the real-time measurements of whichever vital sign category inferred or predicted by the deep learning neural network to be particularly relevant to the medical patient fail to satisfy the customized threshold value inferred or predicted by the deep learning neural network for the medical patient, again hence the term "patient-tailored."

In any case, the patient-tailored GUI can be considered a clinical GUI that is substantively or aesthetically customized or adapted to the clinical idiosyncrasies (e.g., to the unique physical attributes, to the unique medical history) of the medical patient. Contrast this with existing techniques, whose clinical GUIs merely display standardized vital sign measurements in a standardized arrangement or format for every medical patient, no matter their clinical idiosyncrasies.

To help cause the one or more GUI-related inferences described herein to be accurate, the deep learning neural network can first undergo training. In various aspects, the computerized tool described herein can facilitate such training in any suitable fashion (e.g., in supervised fashion) based on any suitable training dataset.

Various embodiments described herein can be employed to use hardware or software to solve problems that are highly technical in nature (e.g., to facilitate intelligent clinical user interfaces), that are not abstract and that cannot be performed as a set of mental acts by a human. Further, some of the processes performed can be performed by a specialized computer (e.g., deep learning neural network having internal parameters such as convolutional kernels) for carrying out defined acts related to clinical user interfaces. For example, such defined acts can include: accessing, by a device operatively coupled to a processor, attribute data corresponding to a medical patient and real-time measurements of a plurality of vital sign categories of the medical patient; identifying, by the device and via execution of a machine learning model on the attribute data, which of the plurality of vital sign categories is clinically relevant to the medical patient, thereby yielding an identified vital sign category; and visually rendering, by the device and on a graphical user interface, real-time measurements of the identified vital sign category. In various aspects, such defined acts can further include determining, by the device and via the execution of the machine learning model, a visual style that is predicted to aid the medical patient's viewing of the graphical user interface, wherein the real-time measurements of the identified vital sign category can be visually rendered according to the visual style. Further still, such defined acts can include: generating, by the device, an electronic alert, in response to the real-time measurements of the identified vital sign category failing to satisfy a threshold value, wherein the threshold value can be computed by the machine learning model.

Such defined acts are not performed manually by humans. Indeed, neither the human mind nor a human with pen and paper can: electronically execute an artificial neural network on a patient's metadata, so as to cause the artificial neural network to identify which of a plurality of vital sign categories (e.g., heart rate category, blood pressure category) is most clinically relevant to the patient, to determine how real-time measurements of such identified vital sign category should be visually stylized (e.g., in terms of font size or color scheme) to ease or improve the patient's viewing comfort, and to compute a unique numerical threshold that separates concerning from non-concerning behavior of the real-time measurements of the identified vital sign category; and electronically render a GUI that displays such real-time measurements of the identified vital sign category according to the determined visual style and that displays alert notifications when such real-time measurements fail to satisfy the computed threshold. Indeed, a machine learning model (e.g., an artificial neural network) is an inherently-computerized construct that simply cannot be meaningfully executed or trained in any way by the human mind without computers. Similarly, a GUI is an inherently-computerized construct that is electronically rendered or projected onto a computer screen and that cannot be meaningfully implemented in any way by the human mind without computers. Accordingly, a computerized tool that can electronically render a clinical GUI whose substantive or aesthetic content is automatically tailored or customized to a patient by a machine learning model is likewise inherently-computerized and cannot be implemented in any sensible, practical, or reasonable way without computers.

Moreover, various embodiments described herein can integrate into a practical application various teachings relating to intelligent clinical user interfaces. As described above, existing techniques generate clinical GUIs in a static, standardized fashion. That is, the clinical GUIs of such existing techniques display real-time measurements of the same, uniform vital sign categories in the same, uniform visual format or arrangement, for all patients. Unfortunately, although such uniform vital sign visualization might be comfortable, appropriate, or relevant for some patients, it is often not comfortable, appropriate, or relevant for all patients.

As the present inventor recognized, such shortcomings of existing techniques can be ameliorated by leveraging artificial intelligence. In particular, various embodiments described herein can involve configuring a deep learning neural network to receive as input metadata (e.g., physical characteristics, demographics, medical history) pertaining to any given medical patient and to produce as output one or more GUI-related inferences regarding that given medical patient. In some aspects, such one or more GUI-related inferences can include a determination as to which of a defined set of vital sign categories is most clinically relevant to the given medical patient (e.g., different vital sign categories might be more or less pertinent to different patients having different pathologies or different physical attributes). In other aspects, such one or more GUI-related inferences can include a determination as to what visual style would help to improve or facilitate the viewing comfort of the given medical patient (e.g., different patients having different pathologies or different physical attributes might find different visual styles more or less appealing or jarring). In yet other aspects, such one or more GUI-related inferences can include computation of a numerical bound that uniquely distinguishes healthy, non-concerning, or acceptable vital sign activity from unhealthy, concerning, or unacceptable vital sign activity. In various instances, various embodiments described herein can further include visually rendering a clinical GUI that is based on or otherwise in compliance with such one or more GUI-related inferences (e.g., that depicts real-time measurements of whichever vital sign category is predicted by the deep learning neural network to be most clinically relevant to the given medical patient; that utilizes the visually stylistic elements predicted by the deep learning neural network to enhance viewing comfort of the given medical patient; that depicts an alert when the real-time measurements of the clinically relevant vital sign category fail to satisfy the unique threshold computed by the deep learning neural network for the given medical patient). In this way, various embodiments described herein can be considered as leveraging artificial intelligence to automatically generate personalized or bespoke clinical GUIs (e.g., personalized or bespoke vital sign visualizations) based on the clinical uniqueness of individual medical patients. In stark contrast, the clinical GUIs of existing techniques display uniform vital sign information using uniform aesthetics without regard to the clinical uniqueness of individual medical patients.

For at least these reasons, various embodiments described herein facilitate improved clinical GUIs, as compared to existing techniques. Thus, various embodiments described herein certainly constitute a tangible and concrete technical improvement or technical advantage in the field of clinical user interfaces. Accordingly, such embodiments clearly qualify as useful and practical applications of computers.

Furthermore, various embodiments described herein can control real-world tangible devices based on the disclosed teachings. For example, various embodiments described herein can electronically train or execute real-world deep learning neural networks on real-world patient metadata, and can electronically render real-world clinical GUIs on real-world computer screens based on the inferencing results produced by such real-world deep learning neural networks.

It should be appreciated that the herein figures and description provide non-limiting examples of various embodiments and are not necessarily drawn to scale.

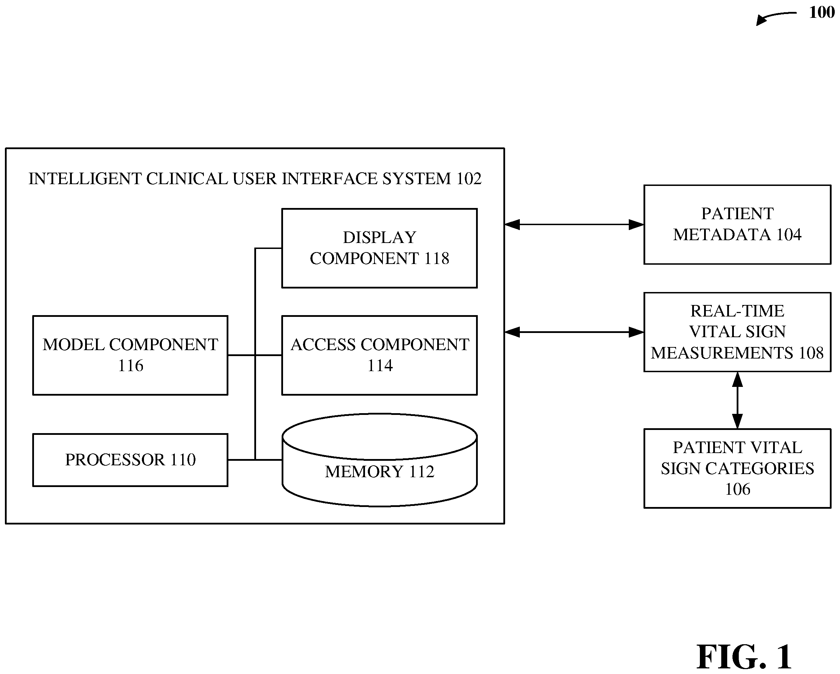

FIG. 1 illustrates a block diagram of an example, non-limiting system 100 that can facilitate intelligent clinical user interfaces in accordance with one or more embodiments described herein. As shown, an intelligent clinical user interface system 102 can be electronically integrated, via any suitable wired or wireless electronic connections, with patient metadata 104 or with a plurality of real-time vital sign measurements 108.

In various embodiments, the patient metadata 104 can correspond to or be associated with any suitable medical patient (e.g., human, animal, or otherwise). In various aspects, the patient metadata 104 can be one or more scalars, one or more vectors, one or more matrices, one or more tensors, one or more character strings, or any suitable combination thereof that indicates, conveys, or otherwise represents any suitable attributes or characteristics of the medical patient. As a non-limiting example, the patient metadata 104 can indicate, convey, or otherwise represent an age or age range of the medical patient. As another non-limiting example, the patient metadata 104 can indicate, convey, or otherwise represent a biological sex or gender of the medical patient. As even another non-limiting example, the patient metadata 104 can indicate, convey, or otherwise represent a body weight or body mass of the medical patient. As yet another non-limiting example, the patient metadata 104 can indicate, convey, or otherwise represent a body height or body width of the medical patient. As still another non-limiting example, the patient metadata 104 can indicate, convey, or otherwise represent a body fat percentage of the medical patient. As another non-limiting example, the patient metadata 104 can indicate, convey, or otherwise represent one or more health habits or lifestyle habits of the medical patient, such as: how much or what types, if any, of tobacco the medical patient uses on a daily, weekly, monthly, or yearly basis; how much or what types, if any, of physical exercise the medical patient performs on a daily, weekly, monthly, or yearly basis; or how much or what types, if any, of alcohol the medical patient consumes on a daily, weekly, monthly, or yearly basis. As yet another non-limiting example, the patient metadata 104 can indicate, convey, or otherwise represent one or more health statuses of the medical patient, such as: whether or not the medical patient is pregnant; how far along in pregnancy the medical patient is; whether or not the medical patient has a known pathology (e.g., cancer, heart disease, diabetes, asthma, COVID-19, glaucoma); or how severe such known pathology is (e.g., metastasized versus non-metastasized cancer). As another non-limiting example, the patient metadata 104 can indicate, convey, or otherwise represent one or more prescribed treatments of the medical patient, such as: what types, if any, of medications the medical patient consumes; what types, if any, of physical therapy or chemotherapy the medical patient undergoes; or what types, if any, of medical operations or surgical interventions that the medical patient has received. As yet another non-limiting example, the patient metadata 104 can indicate, convey, or otherwise represent one or more previously-captured medical images of the medical patient, such as: previously-captured X-ray images of the medical patient's anatomy; previously-captured magnetic resonance images of the medical patient's anatomy; or previously-captured computed tomography images of the medical patient's anatomy. As still another non-limiting example, the patient metadata 104 can indicate, convey, or otherwise represent one or more prostheses or implants that the medical patient possesses, such as: prosthetic limbs; a pacemaker; intravenous stents; or spinal rods. As even another non-limiting example, the patient metadata 104 can indicate, convey, or otherwise represent one or more symptoms of which the medical patient complains, such as: shortness of breath; exercise intolerance; or chest pains. As another non-limiting example, the patient metadata 104 can indicate, convey, or otherwise represent an occupation or profession of the medical patient, such as: office worker; truck driver; or coal miner. As yet another non-limiting example, the patient metadata 104 can indicate, convey, or otherwise represent an education level of the medical patient, such as: primary school; secondary school; undergraduate school; or post-graduate school. Accordingly, the patient metadata 104 can be considered as being or comprising at least a portion of a unique medical history of the medical patient. In other words, the patient metadata 104 can be considered as representing various clinical idiosyncrasies of the medical patient. In various cases, the patient metadata 104 can be typed into or otherwise accessed by any suitable computing device having any suitable human-computer interface, such as: a desktop computer; a laptop computer; a smart phone; or a tablet device.

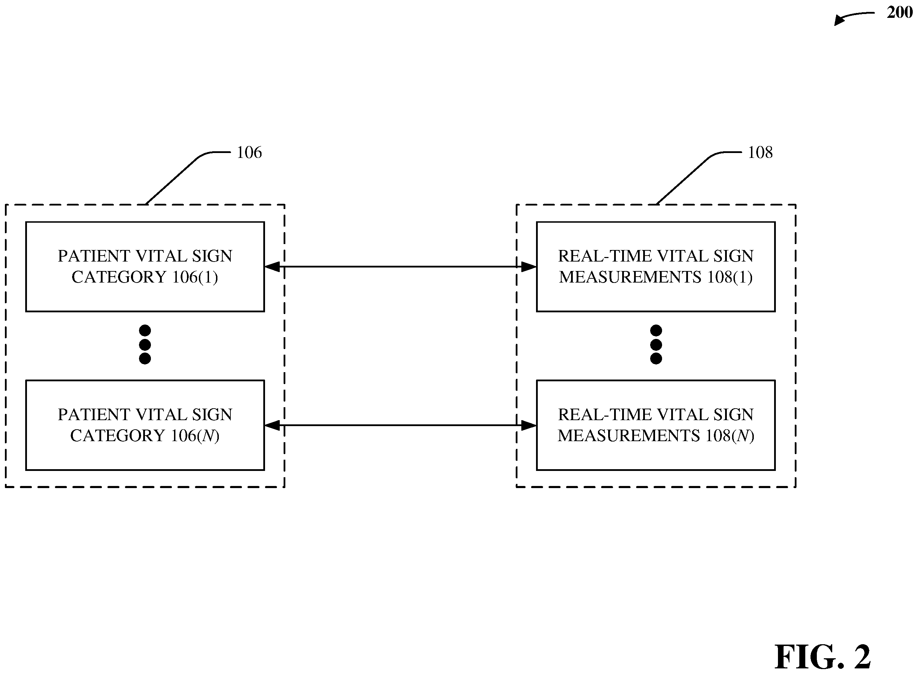

In various embodiments, the medical patient can be considered as being associated with a plurality of patient vital sign categories 106. In various aspects, the plurality of patient vital sign categories 106 can comprise any suitable number of patient vital sign categories, where a patient vital sign category can be any suitable distinct type or class of measurable biological vitality signal. In various instances, the plurality of real-time vital sign measurements 108 can respectively correspond to the plurality of patient vital sign categories 106. Non-limiting aspects are described with respect to FIG. 2.

FIG. 2 illustrates an example, non-limiting block diagram 200 showing the plurality of patient vital sign categories 106 and the plurality of real-time vital sign measurements 108 in accordance with one or more embodiments described herein.

In various aspects, as shown, the plurality of patient vital sign categories 106 can comprise *n* categories, for any suitable positive integer *n:* a patient vital sign category 106(1) to a patient vital sign category 106(*n*). In various instances, each of the plurality of patient vital sign categories 106 can be one or more scalars, one or more vectors, one or more matrices, one or more tensors, one or more character strings, or any suitable combination thereof that can indicate, convey, or otherwise represent a unique or distinct type or class of quantifiable anatomical health or vitality signal of the medical patient. As a non-limiting example, one of the plurality of patient vital sign categories 106 can be a blood pressure category, which can refer to a fluid pressure exhibited by veins or arteries of the medical patient. As another non-limiting example, one of the plurality of patient vital sign categories 106 can be a pulse rate category, which can refer to a rate at which a heart of the medical patient beats. As yet another non-limiting example, one of the plurality of patient vital sign categories 106 can be a pulse strength category, which can refer to a perfusion index exhibited by the heart of the medical patient. As still another non-limiting example, one of the plurality of patient vital sign categories 106 can be an electrocardiogram category, which can refer to cardiac muscle depolarization and repolarization exhibited by the heart of the medical patient. As even another non-limiting example, one of the plurality of patient vital sign categories 106 can be a seismocardiogram category, which can refer to myocardial-induced chest wall acceleration and deceleration exhibited by the medical patient. As another non-limiting example, one of the plurality of patient vital sign categories 106 can be a phonocardiogram category, which can refer to sounds or murmurs exhibited by the heart of the medical patient. As yet another non-limiting example, one of the plurality of patient vital sign categories 106 can be a body temperature category, which can refer to a temperature exhibited by any suitable anatomical structure of the medical patient (e.g., oral temperature, rectal temperature, axillary temperature, ear temperature, skin temperature). As still another non-limiting example, one of the plurality of patient vital sign categories 106 can be a respiration rate category, which can refer to the rate at which the medical patient breathes. As even another non-limiting example, one of the plurality of patient vital sign categories 106 can be an endoscope category, which can refer to endoscopic visual monitoring of any suitable anatomical structure of the medical patient (e.g., an orally-inserted endoscope can visually monitor a trachea, a lung, an esophagus, or a stomach of the medical patient; an anally-inserted endoscope can visually monitor a rectum, a small intestine, or a large intestine of the medical patient; a nasally-inserted endoscope can visually monitor a sinus cavity of the medical patient). As another non-limiting example, one of the plurality of patient vital sign categories 106 can be a blood sugar category, which can refer to the concentration of glucose in blood of the medical patient.

In various cases, as shown, the plurality of real-time vital sign measurements 108 can respectively correspond to the plurality of patient vital sign categories 106. Accordingly, since the plurality of patient vital sign categories 106 can comprise n categories, the plurality of real-time vital sign measurements 108 can likewise comprise n collections of measurements: real-time vital sign measurements 108(1) to real-time vital sign measurements 108(n). In various aspects, each of the plurality of real-time vital sign measurements 108 can be one or more scalars, one or more vectors, one or more matrices, one or more tensors, one or more character strings, or any suitable combination thereof that represent a timeseries of electronically measured data points corresponding to a respective one of the plurality of patient vital sign categories 106.

As a non-limiting example, the real-time vital sign measurements 108(1) can correspond to the patient vital sign category 106(1). Accordingly, the real-time vital sign measurements 108(1) can be a currently-being-recorded timeseries of electronically measured data points associated with the medical patient and that belong to whatever unique or distinct type or class of quantifiable anatomical health or vitality signal is represented by the patient vital sign category 106(1). For instance, suppose that the patient vital sign category 106(1) is a blood pressure category. In such case, the real-time vital sign measurements 108(1) can be a timeseries showing how an electronically-measured blood pressure of the medical patient is currently behaving with respect to time. In another instance, suppose that the patient vital sign category 106(1) is a pulse rate category. In such case, the real-time vital sign measurements 108(1) can be a timeseries showing how an electronically-measured pulse rate of the medical patient is currently behaving with respect to time. In yet another instance, suppose that the patient vital sign category 106(1) is a pulse strength category. In such case, the real-time vital sign measurements 108(1) can be a timeseries showing how an electronically-measured pulse strength (e.g., expressed as perfusion index values) of the medical patient is currently behaving with respect to time. In still another instance, suppose that the patient vital sign category 106(1) is an electrocardiogram category. In such case, the real-time vital sign measurements 108(1) can be an electrocardiogram trace showing how the cardiac muscle of the medical patient is currently polarizing or depolarizing with respect to time. In even another instance, suppose that the patient vital sign category 106(1) is a seismocardiogram category. In such case, the real-time vital sign measurements 108(1) can be a seismocardiogram trace showing how the chest wall of the medical patient is currently accelerating or decelerating with respect to time. In another instance, suppose that the patient vital sign category 106(1) is a phonocardiogram category. In such case, the real-time vital sign measurements 108(1) can be a phonocardiogram trace showing what noises the heart of the medical patient is currently making with respect to time. In yet another instance, suppose that the patient vital sign category 106(1) is a body temperature category. In such case, the real-time vital sign measurements 108(1) can be a timeseries showing how an anatomical temperature of the medical patient is currently behaving with respect to time. In still another instance, suppose that the patient vital sign category 106(1) is an endoscope category. In such case, the real-time vital sign measurements 108(1) can be a currently-being-recorded video-feed produced by an endoscope inserted in the medical patient. In even another instance, suppose that the patient vital sign category 106(1) is a blood sugar category. In such case, the real-time vital sign measurements 108(1) can be a timeseries showing how an electronically-measured blood glucose level of the medical patient is currently behaving with respect to time.

As another non-limiting example, the real-time vital sign measurements 108(n) can correspond to the patient vital sign category 106(n). Thus, just as above, the real-time vital sign measurements 108(n) can be a currently-being-recorded timeseries of electronically measured data points associated with the medical patient and that belong to whatever unique or distinct type or class of quantifiable anatomical health or vitality signal is represented by the patient vital sign category 106(*n*).

In various aspects, any suitable medical diagnostic equipment or sensors can be affixed to, inserted into, or worn by the medical patient so as to electronically capture, record, log, document, or otherwise measure the plurality of real-time vital sign measurements 108. As some non-limiting examples, such equipment or sensors can include: blood pressure gauges; pulse trackers; pulse oximeters; electrocardiogram monitors; seismocardiogram monitors; phonocardiogram monitors; clinical thermometers; clinical endoscopes; or clinical blood glucose monitors.

Referring back to FIG. 1, it can be desired to generate a clinical GUI that displays at least some of the plurality of real-time vital sign measurements 108 in a way that is customized or tailored to the medical patient. As described herein, the intelligent clinical user interface system 102 can facilitate such generation.

In various embodiments, the intelligent clinical user interface system 102 can comprise a processor 110 (e.g., computer processing unit, microprocessor) and a non-transitory computer-readable memory 112 that is operably or operatively or communicatively connected or coupled to the processor 110. The non-transitory computer-readable memory 112 can store computer-executable instructions which, upon execution by the processor 110, can cause the processor 110 or other components of the intelligent clinical user interface system 102 (e.g., access component 114, model component 116, display component 118) to perform one or more acts. In various embodiments, the non-transitory computer-readable memory 112 can store computer-executable components (e.g., access component 114, model component 116, display component 118), and the processor 110 can execute the computer-executable components.

In various embodiments, the intelligent clinical user interface system 102 can comprise an access component 114. In various aspects, the access component 114 can electronically receive or otherwise electronically access the patient metadata 104 or the plurality of real-time vital sign measurements 108. In various instances, the access component 114 can electronically retrieve the patient metadata 104 or the plurality of real-time vital sign measurements 108 from any suitable centralized or decentralized data structures (not shown) or from any suitable centralized or decentralized computing devices (not shown), whether local to or remote from the access component 114. As a non-limiting example, the access component 114 can electronically retrieve the patient metadata 104 from whatever computing devices (e.g., desktop computer, laptop computer, smart phone, tablet) that are responsible for maintaining, storing, or collecting the patient metadata 104. As another non-limiting example, the access component 114 can electronically retrieve the plurality of real-time vital sign measurements 108 from whatever medical diagnostic equipment (e.g., pulse monitors, electrocardiogram monitors, endoscopes) that are used to measure, capture, or create the plurality of real-time vital sign measurements 108. In any case, the access component 114 can electronically obtain or access the patient metadata 104 or the plurality of real-time vital sign measurements 108, such that other components of the intelligent clinical user interface system 102 can electronically interact (e.g., by proxy) with the patient metadata 104 or with the plurality of real-time vital sign measurements 108.

In various embodiments, the intelligent clinical user interface system 102 can comprise a model component 116. In various aspects, the model component 116 can, as described herein, execute a deep learning neural network on the patient metadata 104, thereby yielding various GUI-related inferences pertaining to the medical patient.

In various embodiments, the intelligent clinical user interface system 102 can comprise a display component 118. In various instances, the display component 118 can, as described herein, visually render a clinical GUI that is tailored to the medical patient, based on the various GUI-related inferences produced by the deep learning neural network.

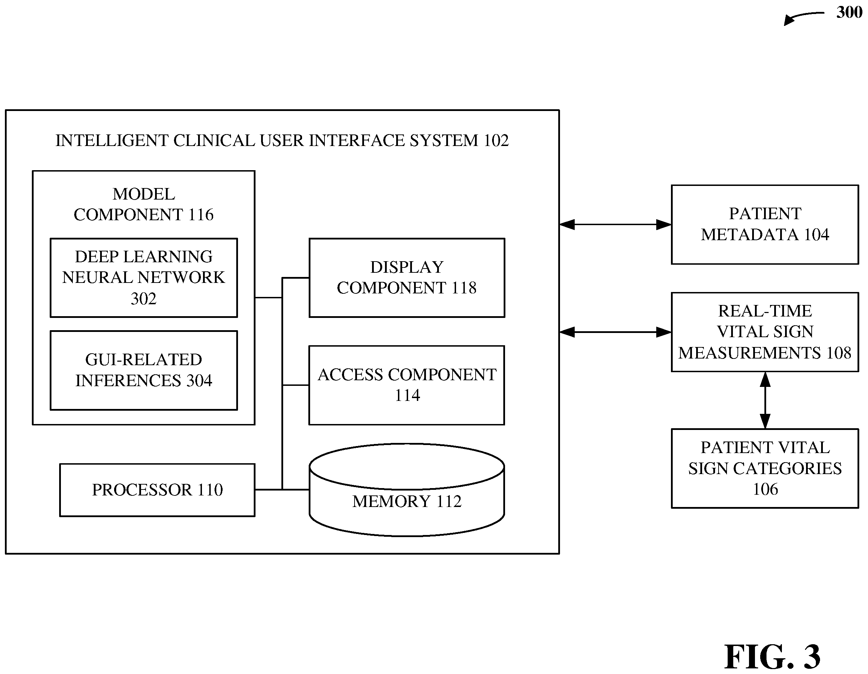

FIG. 3 illustrates a block diagram of an example, non-limiting system 300 including a deep learning neural network and one or more GUI-related inferences that can facilitate intelligent clinical user interfaces in accordance with one or more embodiments described herein. As shown, the system 300 can, in some cases, comprise the same components as the system 100, and can further comprise a deep learning neural network 302 and one or more GUI-related inferences 304.

In various embodiments, the model component 116 can electronically store, electronically maintain, electronically control, or otherwise electronically access the deep learning neural network 302. In various instances, the deep learning neural network 302 can have or otherwise exhibit any suitable deep learning internal architecture. For instance, the deep learning neural network 302 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

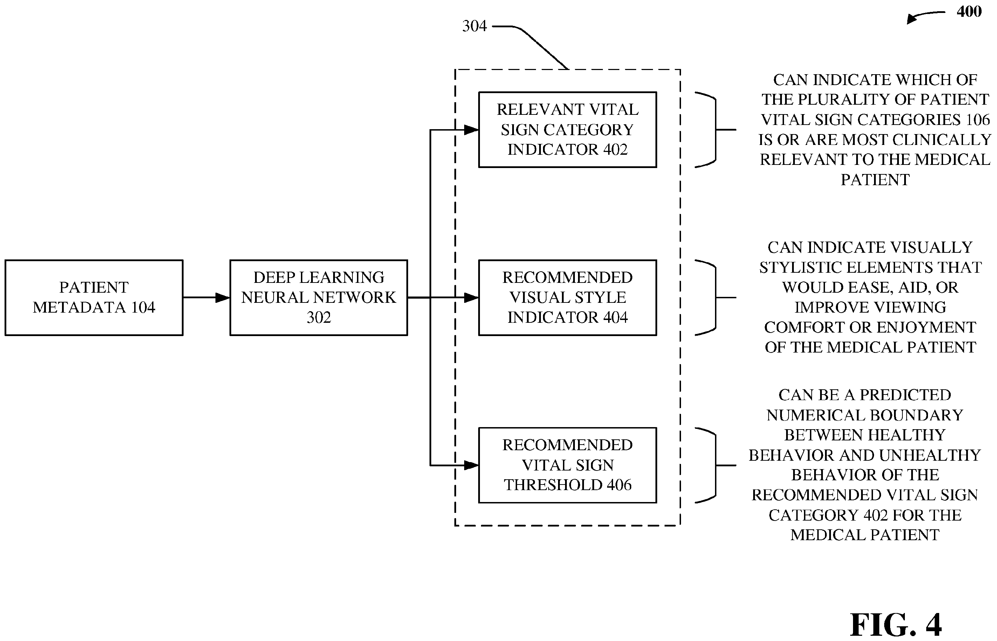

In various aspects, the deep learning neural network 302 can be configured to make various determinations that are pertinent to clinical GUIs, based on inputted medical data. Accordingly, in various instances, the model component 116 can electronically execute the deep learning neural network 302 on the patient metadata 104, and such execution can cause the deep learning neural network 302 to generate the one or more GUI-related inferences 304. Various non-limiting aspects are described with respect to FIG. 4.

FIG. 4 illustrates an example, non-limiting block diagram 400 showing how the deep learning neural network 302 can generate the one or more GUI-related inferences 304 in accordance with one or more embodiments described herein.

In various embodiments, the model component 116 can electronically execute the deep learning neural network 302 on the patient metadata 104. In various aspects, such execution can yield the one or more GUI-related inferences 304. More specifically, the model component 116 can feed the patient metadata 104 to an input layer of the deep learning neural network 302. In various instances, the patient metadata 104 can complete a forward pass through one or more hidden layers of the deep learning neural network 302. In various cases, an output layer of the deep learning neural network 302 can calculate or compute the one or more GUI-related inferences 304, based on activation maps provided by the one or more hidden layers.

In various aspects, the one or more GUI-related inferences 304 can be any suitable electronic data exhibiting any suitable format, size, or dimensionality and indicating, conveying, or otherwise specifying any suitable substantive GUI content or any suitable aesthetic GUI format or arrangement that would be well-suited for, comfortable for, or otherwise relevant to the medical patient. In other words, the patient metadata 104 can be considered as representing the clinical idiosyncrasies (e.g., the unique attributes, the unique medical history) of the medical patient, and the deep learning neural network 302 can be considered as inferring or predicting what content would be appropriate to visually display to the medical patient on a clinical GUI or how that content should be visually shown, so as to best comport or align with those clinical idiosyncrasies.

In various aspects, the one or more GUI-related inferences 304 can comprise a relevant vital sign category indicator 402. In various instances, the relevant vital sign category indicator 402 can be one or more scalars, one or more vectors, one or more matrices, one or more tensors, one or more character strings, or any suitable combination thereof that indicate, convey, or otherwise specify which one of the plurality of patient vital sign categories 106 is most clinically relevant or pertinent to the medical patient. In other words, the patient metadata 104 can represent the clinical idiosyncrasies of the medical patient, and the relevant vital sign category indicator 402 can specify which one of the plurality of patient vital sign categories 106 is predicted by the deep learning neural network 302 as being particularly worthy of being monitored on a clinical GUI when given such clinical idiosyncrasies. In some aspects, the relevant vital sign category indicator 402 can be considered as a segmentation mask that specifies an inferred or predicted relevancy score (e.g., a scalar between 0 and 1, with 0 representing no relevancy and with 1 representing maximum relevancy) for each of the plurality of patient vital sign categories 106. In some cases, whichever one of the plurality of patient vital sign categories 106 has a highest available relevancy score can be considered as being most clinically relevant or pertinent to the medical patient.

Although the herein disclosure mainly describes the relevant vital sign category indicator 402 as specifying or indicating a single one of the plurality of patient vital sign categories 106, this is a mere non-limiting example for ease of explanation. In various aspects, the relevant vital sign category indicator 402 can specify or indicate any suitable number (e.g., one or more) of clinically-relevant patient vital sign categories. For instance, in some cases, the relevant vital sign category indicator 402 can be considered as a segmentation mask that specifies an inferred or predicted relevancy score for each of the plurality of patient vital sign categories 106, and whichever of the plurality of patient vital sign categories 106 have inferred or predicted relevancy scores above any suitable threshold value can be considered as being clinically relevant to the medical patient. As another instance, in some cases, the relevant vital sign category indicator 402 can be considered as a segmentation mask that specifies an inferred or predicted relevancy score for each of the plurality of patient vital sign categories 106, and whichever of the plurality of patient vital sign categories 106 correspond to the top x inferred or predicted relevancy scores, for any suitable positive integer x, can be considered as being clinically relevant to the medical patient.

In any case, the relevant vital sign category indicator 402 can be considered as identifying or designating a patient vital sign category from the plurality of patient vital sign categories 106 that the deep learning neural network 302 has determined is particularly clinically relevant to the patient metadata 104 and thus to the medical patient. After all, different medical patients can have different clinical idiosyncrasies (e.g., different pathologies, different pathology severities, different medications, different lifestyles, different occupational risks), and so different vital sign categories can be considered as being more clinically relevant or less clinically relevant to such different medical patients.

For the purpose of illustrating various non-limiting examples, suppose that the plurality of patient vital sign categories 106 comprises the following: a blood sugar category; a pulse rate category; a body temperature category; a phonocardiogram category; and an intestinal endoscope category. In other words, suppose that the medical patient (e.g., who might currently be in a hospital bed) is currently outfitted with: a blood sugar monitor; a heart rate monitor; a body thermometer; a phonocardiogram monitor; and an intestinal endoscope.

Now, suppose that the patient metadata 104 indicates that the medical patient suffers from diabetes. In such case, the deep learning neural network 302 can infer or predict that blood sugar is more clinically relevant to the medical patient, as compared to pulse rate, body temperature, phonocardiogram, or intestinal endoscope. After all, although it can be considered as wise to monitor all of blood sugar, pulse rate, body temperature, phonocardiogram, or intestinal endoscope for the medical patient, diabetes is a particular pathology that uniquely affects or is affected by blood sugar (e.g., individuals with diabetes are at higher risk of hypoglycemia or hyperglycemia). Thus, the relevant vital sign category indicator 402 can indicate, specify, or designate the blood sugar category as being clinically relevant to the medical patient.

In another instance, suppose that the patient metadata 104 instead indicates that the medical patient has suffered three heart attacks in the previous two years. In such case, the deep learning neural network 302 can infer or predict that pulse rate is more clinically relevant to the medical patient, as compared to blood sugar, body temperature, phonocardiogram, or intestinal endoscope. After all, although it can be considered as wise to monitor all of blood sugar, pulse rate, body temperature, phonocardiogram, or intestinal endoscope for the medical patient, an extensive heart attack history suggests that the medical patient is uniquely prone to unsteady or erratic heart rates (e.g., individuals with many heart attacks are at higher risk of tachycardia or bradycardia). Thus, the relevant vital sign category indicator 402 can indicate or specify the pulse rate category as being clinically relevant to the medical patient.

In yet another instance, suppose that the patient metadata 104 instead indicates that the medical patient is on a trazodone regimen. In such case, the deep learning neural network 302 can infer or predict that body temperature is more clinically relevant to the medical patient, as compared to blood sugar, pulse rate, phonocardiogram, or intestinal endoscope. After all, although it can be considered as wise to monitor all of blood sugar, pulse rate, body temperature, phonocardiogram, or intestinal endoscope for the medical patient, trazodone is a particular medication that is known to cause anatomical temperature fluctuations (e.g., night sweats) as a side effect. Thus, the relevant vital sign category indicator 402 can indicate or specify the body temperature category as being clinically relevant to the medical patient.

In even another instance, suppose that the patient metadata 104 instead indicates that the medical patient has complained of persistent chest pain and coughing. In such case, the deep learning neural network 302 can infer or predict that phonocardiogram is more clinically relevant to the medical patient, as compared to blood sugar, pulse rate, body temperature, or intestinal endoscope. After all, although it can be considered as wise to monitor all of blood sugar, pulse rate, body temperature, phonocardiogram, or intestinal endoscope for the medical patient, persistent chest pain and coughing are symptoms that often accompany heart murmurs or heart valve disease, and phonocardiogram traces are well-suited for diagnosing heart murmurs or heart valve disease. Thus, the relevant vital sign category indicator 402 can indicate or specify the phonocardiogram category as being clinically relevant to the medical patient.

In still another instance, suppose that the patient metadata 104 instead indicates that the medical patient is afflicted with tapeworms. In such case, the deep learning neural network 302 can infer or predict that intestinal endoscope is more clinically relevant to the medical patient, as compared to blood sugar, pulse rate, body temperature, or phonocardiogram. After all, although it can be considered as wise to monitor all of blood sugar, pulse rate, body temperature, phonocardiogram, or intestinal endoscope for the medical patient, tapeworms are parasites that often invade the digestive tract and can thus be viewed or detected by an intestinal endoscope. Thus, the relevant vital sign category indicator 402 can indicate or specify the intestinal endoscope category as being clinically relevant to the medical patient.

In this way, the deep learning neural network 302 can be considered as inferring or predicting which of the plurality of patient vital sign categories 106 would be most helpful, most called for, or otherwise most warranted given the clinical idiosyncrasies represented by the patient metadata 104, and the relevant vital sign category indicator 402 can convey or represent such inference or prediction.

In various aspects, the one or more GUI-related inferences 304 can comprise a recommended visual style indicator 404. In various instances, the recommended visual style indicator 404 can be one or more scalars, one or more vectors, one or more matrices, one or more tensors, one or more character strings, or any suitable combination thereof that indicate, convey, specify, characterize, describe, or otherwise define any suitable visual style that would aid, improve, ease, or otherwise benefit viewing comfort of the medical patient. In other words, the patient metadata 104 can represent the clinical idiosyncrasies of the medical patient, and the recommended visual style indicator 404 can indicate a visual style that is predicted by the deep learning neural network 302 as being comfortably viewable by, appealing to, or otherwise recommended for those with such clinical idiosyncrasies. In various cases, the recommended visual style indicator 404 can be considered as a classification label that identifies any suitable number of any suitable types of visually stylistic elements that are predicted to be comfortably viewed by the medical patient. As a non-limiting example, such visually stylistic elements can include font size. That is, the recommended visual style indicator 404 can specify one or more font sizes to use or to avoid so as to improve or preserve the medical patient's viewing comfort or enjoyment. As another non-limiting example, such visually stylistic elements can include color scheme. That is, the recommended visual style indicator 404 can specify one or more colors or combinations of colors to use or to avoid so as to improve or preserve the medical patient's viewing comfort or enjoyment. As even another non-limiting example, such visually stylistic elements can include brightness or contrast. That is, the recommended visual style indicator 404 can specify one or more screen brightness or contrast levels to use or to avoid so as to improve or preserve the medical patient's viewing comfort or enjoyment. As yet another non-limiting example, such visually stylistic elements can include geometric elements. That is, the recommended visual style indicator 404 can specify one or more geometric or spatial shapes or patterns to use or to avoid so as to improve or preserve the medical patient's viewing comfort or enjoyment.

In any case, the recommended visual style indicator 404 can be considered as identifying a visual style that the deep learning neural network 302 has determined is comfortable or appealing given the patient metadata 104 (and is thus comfortable or appealing to the medical patient). After all, different medical patients can have different clinical idiosyncrasies (e.g., different pathologies, different pathology severities, different medications, different lifestyles, different occupational risks), and different visual styles can thus be more or less tolerable to such different medical patients.

For instance, suppose that the patient metadata 104 indicates that the medical patient is diagnosed with macular degeneration and utilizes eye prescription lens having over three diopters of correction. In such case, the deep learning neural network 302 can infer that the medical patient has very poor eyesight and can predict or infer a minimum font size that the medical patient can view comfortably given their unique macular degeneration and eye prescription. Thus, the recommended visual style indicator 404 can indicate or specify that no font smaller than that predicted or inferred minimum font size should be used when displaying a clinical GUI to the medical patient.

As another instance, suppose that the patient metadata 104 instead indicates that the medical patient is diagnosed with protanopia. In such case, the deep learning neural network 302 can infer that the medical patient is unable to reliably or comfortably discern shades of red. Thus, the recommended visual style indicator 404 can indicate or specify that shades of red should be avoided when displaying a clinical GUI to the medical patient.

As yet another instance, suppose that the patient metadata 104 instead indicates that the medical patient is diagnosed with photic sneezing syndrome. In such case, the deep learning neural network 302 can infer that the medical patient is prone to suddenly sneeze when abruptly exposed to bright lights and can predict or infer a maximum screen brightness level that the medical patient can view comfortably (e.g., without sneezing) given their unique syndrome. Thus, the recommended visual style indicator 404 can indicate or specify that no screen brightness higher than that predicted or inferred maximum brightness level should be used when displaying a clinical GUI to the medical patient.

As even another instance, suppose that the patient metadata 104 instead indicates that the medical patient has a history of photo-induced epileptic seizures. In such case, the deep learning neural network 302 can infer that the medical patient is prone to seizing when exposed to highly alternating geometric patterns, such as checkerboards, stripes, or spirals. Thus, the recommended visual style indicator 404 can indicate or specify that no checkerboard patterns, striped patterns, or spiral patterns should be used when displaying a clinical GUI to the medical patient.

In this way, the deep learning neural network 302 can be considered as inferring or predicting what aesthetic format or visual scheme would be most comfortable, tolerable, or appealing given the clinical idiosyncrasies represented by the patient metadata 104, and the recommended visual style indicator 404 can convey or represent such inference or prediction.

In various aspects, the one or more GUI-related inferences 304 can comprise a recommended vital sign threshold 406. In various instances, the recommended vital sign threshold 406 can one or more scalars, one or more vectors, one or more matrices, one or more tensors, or any suitable combination thereof that indicates or otherwise represents a numerical bound that demarcates, delineates, or defines healthy or acceptable behavior of whichever patient vital sign category is designated by the relevant vital sign category indicator 402. In other words, the patient metadata 104 can represent the clinical idiosyncrasies of the medical patient, the relevant vital sign category indicator 402 can designate which specific vital sign category is most clinically relevant given those clinical idiosyncrasies, and the recommended vital sign threshold 406 can indicate a predicted upper bound (or a predicted lower bound) that measurements for that specific vital sign category should not exceed (or fall beneath) given those clinical idiosyncrasies. That is, the recommended vital sign threshold 406 can be considered as identifying a numerical boundary that the deep learning neural network 302 has determined separates healthy or acceptable vital sign behavior from unhealthy or unacceptable vital sign behavior given the patient metadata 104. After all, different medical patients can have different clinical idiosyncrasies (e.g., different pathologies, different pathology severities, different medications, different lifestyles, differential occupational risks), and what constitutes or qualifies as acceptable vital sign activity can vary across such different medical patients.

As a non-limiting example, suppose that the relevant vital sign category indicator 402 designates blood pressure as the clinically relevant vital sign category, and suppose further that the patient metadata 104 indicates that the medical patient has a history of suffering from hypertension. In such case, the deep learning neural network 302 can infer that the medical patient is prone to having excessively high blood pressure. Accordingly, the deep learning neural network 302 can predict a unique maximum blood pressure value above which the medical patient's blood pressure would be considered as concerning or unhealthy, and the recommended vital sign threshold 406 can be considered as that unique maximum blood pressure value. Note that the conservativeness of that unique maximum blood pressure value can be based on whatever other information is provided in the patient metadata 104. For instance, if the patient metadata 104 indicates that the medical patient is sedentary and obese, then the deep learning neural network 302 can cause that unique maximum blood pressure value to be more conservative (e.g., to be at a lower maximum value since the medical patient's lifestyle suggests that the medical patient is less physically resilient). On the other hand, if the patient metadata 104 indicates that the medical patient is physically active and not obese, then the deep learning neural network 302 can cause that unique maximum blood pressure value to be less conservative (e.g., to be at a higher maximum value since the medical patient's lifestyle suggests that the medical patient is more physically resilient).

As another non-limiting example, suppose that the relevant vital sign category indicator 402 designates blood pressure as the clinically relevant vital sign category, and suppose further that the patient metadata 104 indicates that the medical patient has a history of suffering from hypotension. In such case, the deep learning neural network 302 can infer that the medical patient is prone to having excessively low blood pressure. Accordingly, the deep learning neural network 302 can predict a unique minimum blood pressure value below which the medical patient's blood pressure would be considered as concerning or unhealthy, and the recommended vital sign threshold 406 can be considered as that unique minimum blood pressure value. Just as above, note that the conservativeness of that unique minimum blood pressure value can be based on whatever other information is provided in the patient metadata 104. For instance, if the patient metadata 104 indicates that the medical patient is a habitual smoker, then the deep learning neural network 302 can cause that unique minimum blood pressure value to be more conservative (e.g., to be at a high minimum value since the medical patient's lifestyle suggests that the medical patient is less physically resilient). On the other hand, if the patient metadata 104 indicates that the medical patient is not a habitual smoker, then the deep learning neural network 302 can cause that unique minimum blood pressure value to be less conservative (e.g., to be at a lower minimum value since the medical patient's lifestyle suggests that the medical patient is more physically resilient).

In this way, the deep learning neural network 302 can be considered as inferring or predicting not just which patient vital sign category would be most clinically relevant given the clinical idiosyncrasies represented by the patient metadata 104, but also a numerical bound derived from those clinical idiosyncrasies that delineates healthy or acceptable behavior of that patient vital sign category. The recommended vital sign threshold 406 can convey or represent such numerical bound.

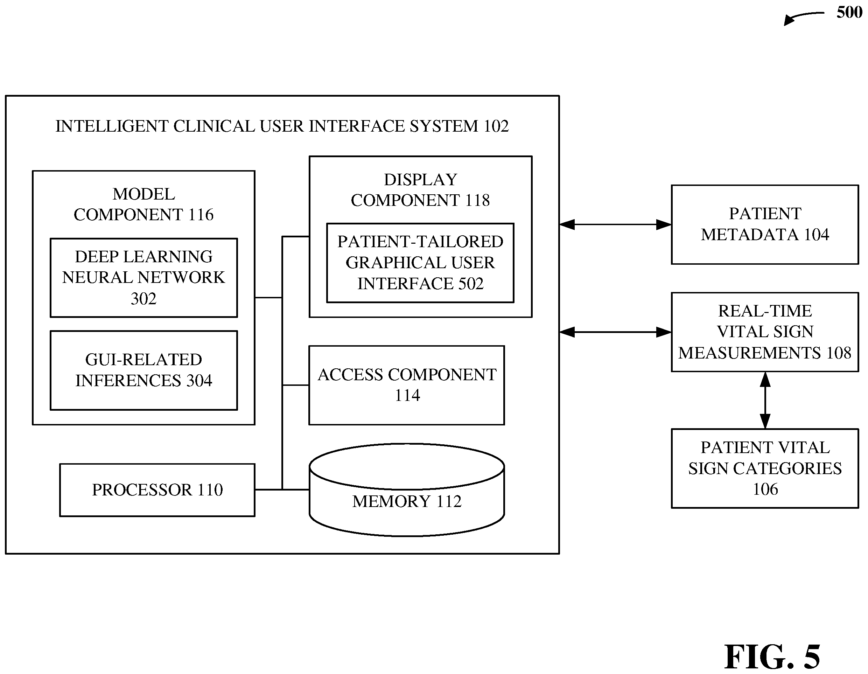

FIG. 5 illustrates a block diagram of an example, non-limiting system 500 including a patient-tailored GUI that can facilitate intelligent clinical user interfaces in accordance with one or more embodiments described herein. As shown, the system 500 can, in some cases, comprise the same components as the system 300, and can further comprise a patient-tailored graphical user interface 502 (hereafter "patient-tailored GUI 502").

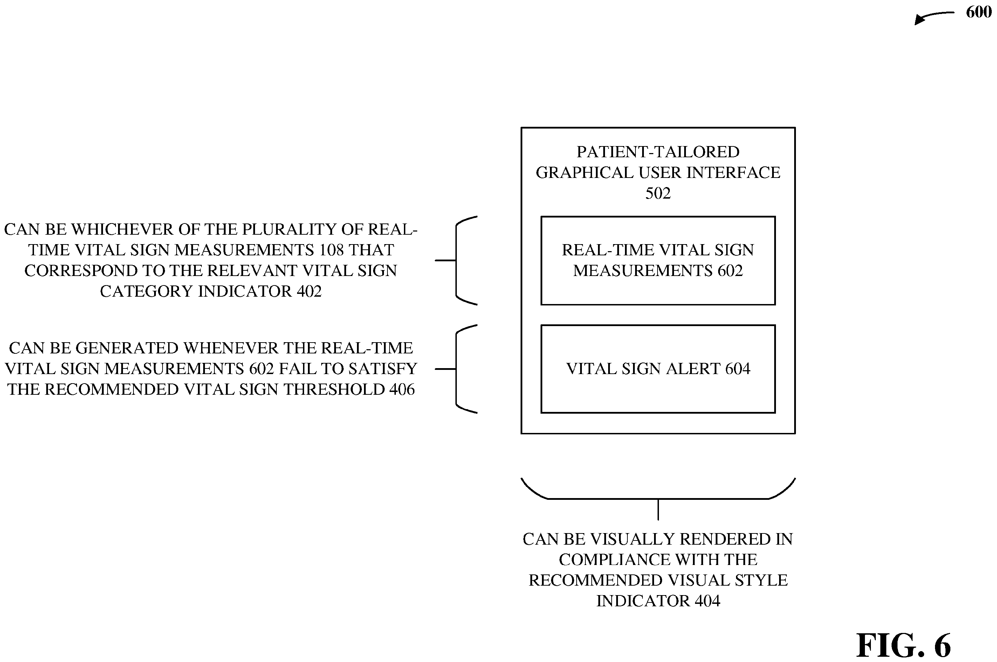

In various embodiments, the display component 118 can electronically generate the patient-tailored GUI 502, based on the one or more GUI-related inferences 304. In various aspects, the display component 118 can visually render, or otherwise cause to be visually rendered, the patient-tailored GUI 502 on any suitable electronic display of any suitable computing device. As a non-limiting example, the display component 118 can cause the patient-tailored GUI 502 to be rendered on an electronic computer screen of any suitable smart phone device (e.g., a smart phone of the medical patient). As another non-limiting example, the display component 118 can cause the patient-tailored GUI 502 to be rendered on an electronic computer screen of any suitable wearable device (e.g., smart watch of the medical patient, smart glasses of the medical patient). As even another non-limiting example, the display component 118 can cause the patient-tailored GUI 502 to be rendered on an electronic computer screen of any suitable hospital console device (e.g., a bedside hospital monitor that is near the medical patient). In any case, the substantive content or aesthetic formatting of the patient-tailored GUI 502 can be based on the one or more GUI-related inferences 304 and can thus be considered as being customized or tailored to the medical patient. Various non-limiting aspects are described with respect to FIG. 6.

FIG. 6 illustrates an example, non-limiting block diagram 600 showing the patient-tailored GUI 502 in accordance with one or more embodiments described herein.

In various embodiments, as shown, the patient-tailored GUI 502 can comprise real-time vital sign measurements 602. In various aspects, as mentioned above, the relevant vital sign category indicator 402 can designate a patient vital sign category from the plurality of patient vital sign categories 106 as being clinically relevant to the medical patient. In various instances, the real-time vital sign measurements 602 can be whichever of the plurality of real-time vital sign measurements 108 corresponds to that designated patient vital sign category. As a non-limiting example, suppose that the relevant vital sign category indicator 402 designates the patient vital sign category 106(1) as being clinically relevant to the medical patient. In such case, the real-time vital sign measurements 108(1) can be considered as the real-time vital sign measurements 602. As another non-limiting example, suppose that the relevant vital sign category indicator 402 instead designates the patient vital sign category 106(*n*) as being clinically relevant to the medical patient. In such case, the real-time vital sign measurements 108(*n*) can be considered as the real-time vital sign measurements 602. In any case, the real-time vital sign measurements 602 can be a timeseries showing the current temporally-varying behavior or activity of the designated patient vital sign category, and the patient-tailored GUI 502 can visually display (e.g., in numerical fashion or in graphical fashion) such timeseries. In other words, the patient-tailored GUI 502 can be considered as visually depicting or illustrating the real-time measurements of whichever vital sign category is predicted or inferred by the deep learning neural network 302 as being most clinically relevant to the medical patient. For at least this reason, the term "patient-tailored" can be considered as appropriate.

In some cases, the patient-tailored GUI 502 can omit or exclude all the rest of the plurality of real-time vital sign measurements 108. However, this is a mere non-limiting example. In other cases, the patient-tailored GUI 502 can display any others of the plurality of real-time vital sign measurements 108 along with the real-time vital sign measurements 602. However, in such cases, those other real-time vital sign measurements can be visually depicted or illustrated in a minimized fashion. As a non-limiting example, those other real-time vital sign measurements can be depicted or illustrated in the patient-tailored GUI 502 using a smaller font size, a smaller graph size, or less noticeable or apparent colors than are used for the real-time vital sign measurements 602. Accordingly, those other real-time vital sign measurements can be depicted or illustrated without significantly drawing attention away from the real-time vital sign measurements 602.

In various aspects, the patient-tailored GUI 502 can be visually rendered according to or otherwise in compliance with the recommended visual style indicator 404. As a non-limiting example, suppose that the recommended visual style indicator 404 specifies a minimum or maximum font size that is predicted to benefit viewing comfort or tolerance of the medical patient. In such case, the patient-tailored GUI 502 can depict or illustrate the real-time vital sign measurements 602 consistent with such minimum or maximum font size. As another non-limiting example, suppose that the recommended visual style indicator 404 specifies color restrictions that are predicted to benefit viewing comfort or tolerance of the medical patient. In such case, the patient-tailored GUI 502 can depict or illustrate the real-time vital sign measurements 602 consistent with such color restrictions. As yet another non-limiting example, suppose that the recommended visual style indicator 404 specifies a minimum or maximum screen brightness level that is predicted to benefit viewing comfort or tolerance of the medical patient. In such case, the patient-tailored GUI 502 can depict or illustrate the real-time vital sign measurements 602 consistent with such minimum or maximum screen brightness level. As still another non-limiting example, suppose that the recommended visual style indicator 404 specifies geometric pattern restrictions that are predicted to benefit viewing comfort or tolerance of the medical patient. In such case, the patient-tailored GUI 502 can depict or illustrate the real-time vital sign measurements 602 consistent with such geometric pattern restrictions. In other words, the patient-tailored GUI 502 can be considered as being visually rendered in whatever aesthetic fashion that the deep learning neural network 302 predicted or inferred would be uniquely comfortable or tolerable for the medical patient. Again, for at least this reason, the term "patient-tailored" can be considered as appropriate.

In various aspects, the patient-tailored GUI 502 can comprise a vital sign alert 604. In various instances, the vital sign alert 604 can be any suitable electronic data (e.g., one or more scalars, one or more vectors, one or more matrices, one or more tensors, or any suitable combination thereof) that indicates, conveys, or otherwise represents that the real-time vital sign measurements 602 exhibit unhealthy behavior or activity. In particular, the display component 118 can electronically compare (e.g., on a continual, periodic, or *ad hoc* basis) the real-time vital sign measurements 602 (or at least the most recent data entry thereof) to the recommended vital sign threshold 406. If the real-time vital sign measurements 602 (or at least the most recent data entry thereof) fails to satisfy the recommended vital sign threshold 406, then the display component 118 can cause the patient-tailored GUI 502 to electronically depict or illustrate the vital sign alert 604. On the other hand, if the real-time vital sign measurements 602 (or at least the most recent data entry thereof) satisfies the recommended vital sign threshold 406, then the display component 118 can instead refrain from causing the patient-tailored GUI 502 to electronically depict or illustrate the vital sign alert 604. In this way, the patient-tailored GUI 502 can electronically warn when whichever vital sign category that is predicted as being clinically relevant to the medical patient deviates from whatever numerical bound that is predicted to distinguish between healthy and unhealthy vital sign activity for the medical patient. Again, for at least this reason, the term "patient-tailored" can be considered as appropriate.

It is to be appreciated that any other suitable aspects or details associated with clinical GUIs can be implemented in conjunction with the patient-tailored GUI 502. As some non-limiting examples, the patient-tailored GUI 502 can implement: any suitable data packaging, analysis, or exporting techniques (e.g., diagnostic models can analyze the real-time vital sign measurements 602); any suitable augmented reality or virtual reality techniques (e.g., if scanned or endoscopic images of the medical patient are available, they can be leveraged to construct a two-dimensional or three-dimensional virtual model of an anatomical structure of the medical patient, and such two-dimensional or three-dimensional model can be superimposed over an image or live feed that depicts the medical patient); any suitable gamification techniques (e.g., reward points can be defined or earned depending upon how much user interaction the patient-tailored GUI 502 receives); any suitable social media or telemedicine techniques (e.g., the real-time vital sign measurements 602 can be streamed to any suitable remote device or electronic social media account); or any suitable preference selection techniques.

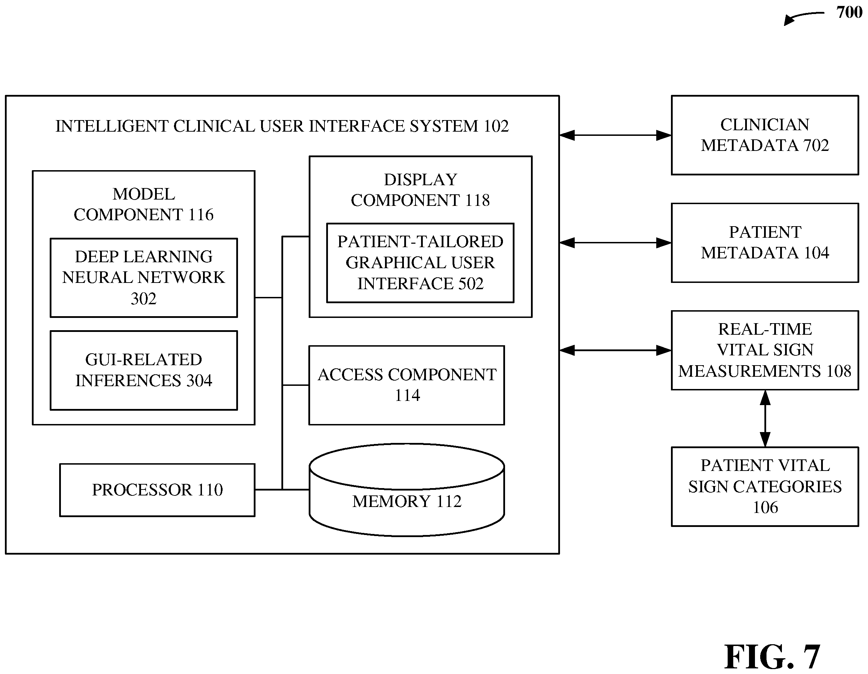

FIG. 7 illustrates a block diagram of an example, non-limiting system 700 including clinician metadata that facilitates intelligent clinical user interfaces in accordance with one or more embodiments described herein. As shown, the system 700 can, in some cases, comprise the same components as the system 500, and can further comprise clinician metadata 702.

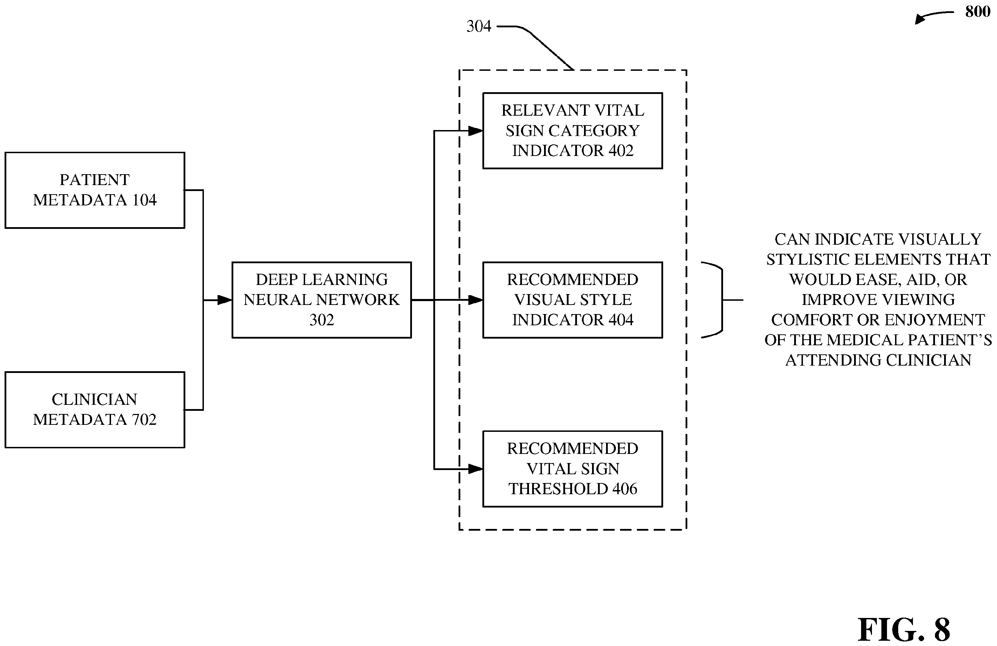

In various embodiments, the clinician metadata 702 can correspond to or be associated with any suitable clinician (e.g., physician, nurse, medical technician) that is attending to, treating, or otherwise associated with the medical patient. In various aspects, the clinician metadata 702 can be one or more scalars, one or more vectors, one or more matrices, one or more tensors, one or more character strings, or any suitable combination thereof that indicates, conveys, or otherwise represents any suitable attributes or characteristics (e.g., age, lifestyle, pathologies, education level, preferences) of the attending clinician. In various instances, the access component 114 can electronically receive, retrieve, or access the clinician metadata 702 from any suitable source. In various cases, the one or more GUI-related inferences 304 can be based on the clinician metadata 702, such that the patient-tailored GUI 502 can be considered as also being tailored to the attributes of the attending clinician. Various non-limiting aspects are described with respect to FIG. 8.

FIG. 8 illustrates an example, non-limiting block diagram 800 showing how the clinician metadata 702 can be taken into account in accordance with one or more embodiments described herein.

In various aspects, rather than executing the deep learning neural network 302 on the patient metadata 104 alone, the model component 116 can instead electronically execute the deep learning neural network 302 on both the patient metadata 104 and the clinician metadata 702. In various instances, such execution can yield the one or more GUI-related inferences 304. More specifically, the model component 116 can concatenate the patient metadata 104 and the clinician metadata 702 together and can feed that concatenation to the input layer of the deep learning neural network 302. In various cases, that concatenation can complete a forward pass through the one or more hidden layers of the deep learning neural network 302. In various aspects, the output layer of the deep learning neural network 302 can calculate or compute the one or more GUI-related inferences 304, based on activation maps provided by the one or more hidden layers.

In various instances, the relevant vital sign category indicator 402 and the recommended vital sign threshold 406 can be as described above. However, in various cases, when the deep learning neural network 302 is executed on the clinician metadata 702, the recommended visual style indicator 404 can be slightly different than as described above. In particular, when the deep learning neural network 302 is executed on the clinician metadata 702, the recommended visual style indicator 404 can indicate or specify visually stylistic elements that would ease, aid, or otherwise improve viewing comfort, enjoyment, or tolerance of not just the medical patient but also of the attending clinician. As some non-limiting examples, the recommended visual style indicator 404 can indicate or specify font sizes, color schemes, brightness levels, contrast levels, or geometric patterns that are predicted by the deep learning neural network 302 to be comfortable or appealing to the attending clinician (e.g., in addition to, or instead of, those that are predicted to be comfortable or appealing to the medical patient). Accordingly, the patient-tailored GUI 502 can, in such cases, be considered as being tailored not just to the medical patient but also to the attending clinician.

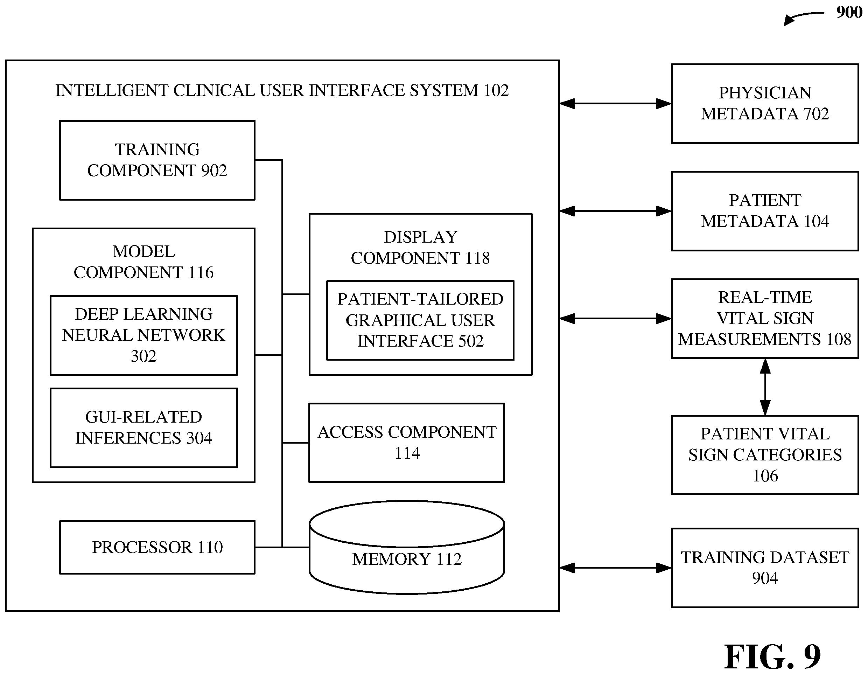

In order for the one or more GUI-related inferences 304 to be accurate, correct, or reliable, the deep learning neural network 302 can first undergo training, as described with respect to FIGs. 9-11.

FIG. 9 illustrates a block diagram of an example, non-limiting system 900 including a training component and a training dataset that can facilitate intelligent clinical user interfaces in accordance with one or more embodiments described herein. As shown, the system 900 can, in some cases, comprise the same components as the system 700, and can further comprise a training component 902 and a training dataset 904.

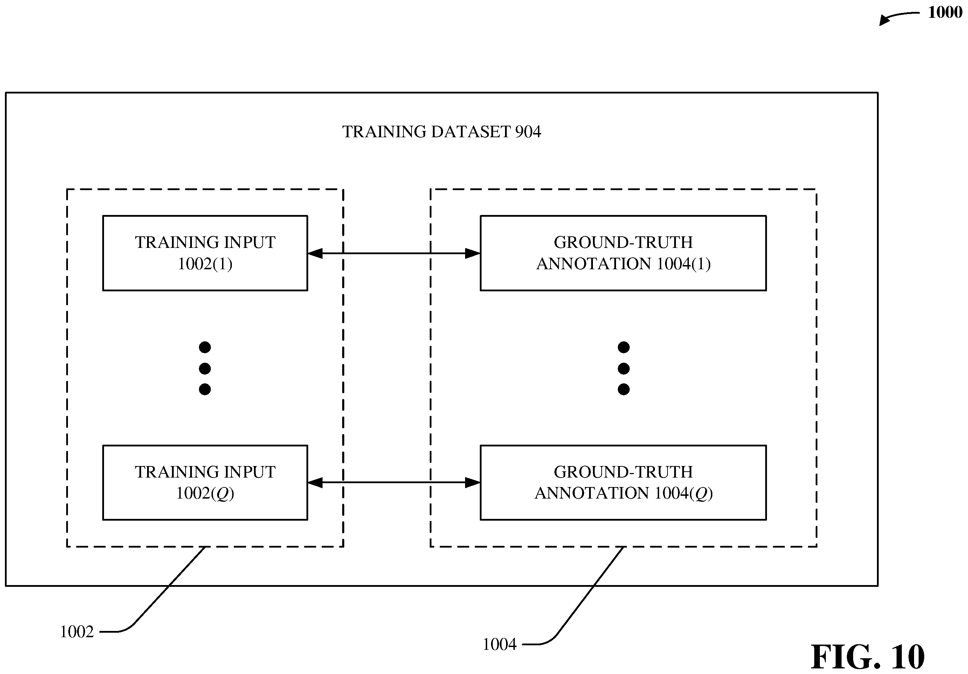

In various aspects, the access component 114 can electronically receive, retrieve, or otherwise access, from any suitable source, the training dataset 904, and the training component 902 can electronically train the deep learning neural network 302 on the training dataset 904. Various non-limiting aspects are described with respect to FIGs. 10-11.

FIG. 10 illustrates an example, non-limiting block diagram 1000 of the training dataset 904 in accordance with one or more embodiments described herein.

In various aspects, the training dataset 904 can comprise a set of training inputs 1002. In various instances, the set of training inputs 1002 can include q inputs for any suitable positive integer q: a training input 1002(1) to a training input 1002(q). In various instances, each of the set of training inputs 1002 can be training patient metadata having the same format, size, or dimensionality as the patient metadata 104. In various other instances, each of the set of training inputs 1002 can be a concatenation between training patient metadata (e.g., having the same format, size, or dimensionality as the patient metadata 104) and training clinician metadata (e.g., having the same format, size, or dimensionality as the clinician metadata 702).

In various aspects, the training dataset 904 can comprise a set of ground-truth annotations 1004 that can respectively correspond to the set of training inputs 1002. Accordingly, since the set of training inputs 1002 can have q inputs, the set of ground-truth annotations 1004 can have q annotations: a ground-truth annotation 1004(1) to a ground-truth annotation 1004(q). In various instances, each of the set of ground-truth annotations 1004 can be one or more correct or accurate GUI-related inferences (e.g., having the same format, size, or dimensionality as the one or more GUI-related inferences 304) that are known or deemed to correspond to a respective one of the set of training inputs 1002. As a non-limiting example, the ground-truth annotation 1004(1) can correspond to the training input 1002(1). Accordingly, the ground-truth annotation 1004(1) can be the correct or accurate GLTI-related inferences (e.g., correct or accurate relevant vital sign category indicator; correct or accurate recommended visual style indicator; correct or accurate recommended vital sign threshold) that are known or deemed to correspond to the training input 1002(1). As another non-limiting example, the ground-truth annotation 1004(*q*) can correspond to the training input 1002(*q*). Accordingly, the ground-truth annotation 1004(*q*) can be the correct or accurate GUI-related inferences that are known or deemed to correspond to the training input 1002(*q*).

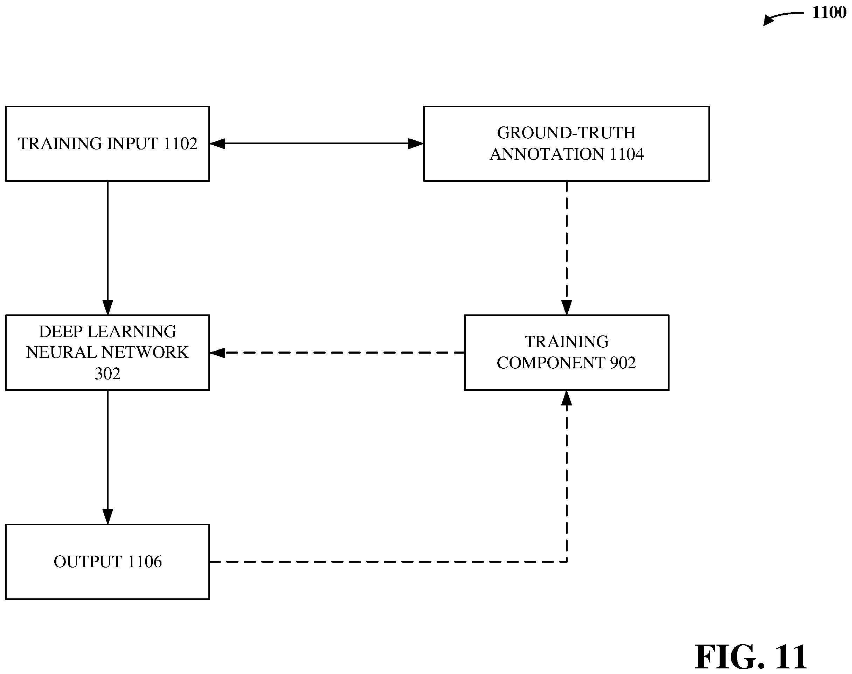

FIG. 11 illustrates an example, non-limiting block diagram 1100 showing how the deep learning neural network 302 can be trained in accordance with one or more embodiments described herein.

In various aspects, prior to beginning training, trainable internal parameters (e.g., convolutional kernels, weight matrices, bias values) of the deep learning neural network 302 can be initialized in any suitable fashion (e.g., via random initialization).

In various aspects, the training component 902 can select a training input 1102 and a ground-truth annotation 1104 corresponding to the training input 1102 from the training dataset 904. In various instances, the training component 902 can execute the deep learning neural network 302 on the training input 1102, thereby causing the deep learning neural network 302 to produce an output 1106. More specifically, in some cases, the training component 902 can feed the training input 1102 to the input layer of the deep learning neural network 302, the training input 1102 can complete a forward pass through the one or more hidden layers of the deep learning neural network 302, and the output layer of the deep learning neural network 302 can compute the output 1106 based on activation maps or feature maps provided by the one or more hidden layers of the deep learning neural network 302.

Note that the format, size, or dimensionality of the output 1106 can be dictated by the number, arrangement, sizes, or other characteristics of the neurons, convolutional kernels, or other internal parameters of the output layer (or of any other layers) of the deep learning neural network 302. Accordingly, the output 1106 can be forced to have any desired format, size, or dimensionality, by adding, removing, or otherwise adjusting characteristics of the output layer (or of any other layers) of the deep learning neural network 302. So, the output 1106 can be considered as the predicted GUI-related inferences (e.g., predicted relevant vital sign category indicator; predicted recommended visual style indicator; predicted recommended vital sign threshold) that the deep learning neural network 302 believes should correspond to the training input 1102. In contrast, the ground-truth annotation 1104 can be considered as the correct or accurate GUI-related inferences (e.g., correct or accurate relevant vital sign category indicator; correct or accurate recommended visual style indicator; correct or accurate recommended vital sign threshold) that is known or deemed to correspond to the training input 1102. Note that, if the deep learning neural network 302 has so far undergone no or little training, then the output 1106 can be highly inaccurate (e.g., can be very different from the ground-truth annotation 1104).

In various aspects, the training component 902 can compute any suitable error or loss (e.g., mean absolute error, mean squared error, cross-entropy error) between the output 1106 and the ground-truth annotation 1104. In various instances, the training component 902 can incrementally update the trainable internal parameters of the deep learning neural network 302, via backpropagation (e.g., stochastic gradient descent) driven by the computed error or loss.

In various cases, such execution-and-update procedure can be repeated for any suitable number of training inputs (e.g., for each training input in the training dataset 904). This can ultimately cause the trainable internal parameters of the deep learning neural network 302 to become iteratively optimized for accurately generating GUI-related inferences (e.g., relevant vital sign category indicators; recommended visual style indicators; recommended vital sign thresholds) based on inputted patient metadata (which may or may not be accompanied by inputted clinician metadata). In various aspects, the training component 902 can implement any suitable training batch sizes, any suitable error, loss, or objective functions, or any suitable training termination criteria.

Although the above description mainly describes the deep learning neural network 302 as being trained in supervised fashion, this is a mere non-limiting example for ease of illustration and explanation. In various cases, the training component 902 can implement any other suitable training paradigms (e.g., unsupervised training, reinforcement learning) to train the deep learning neural network 302.

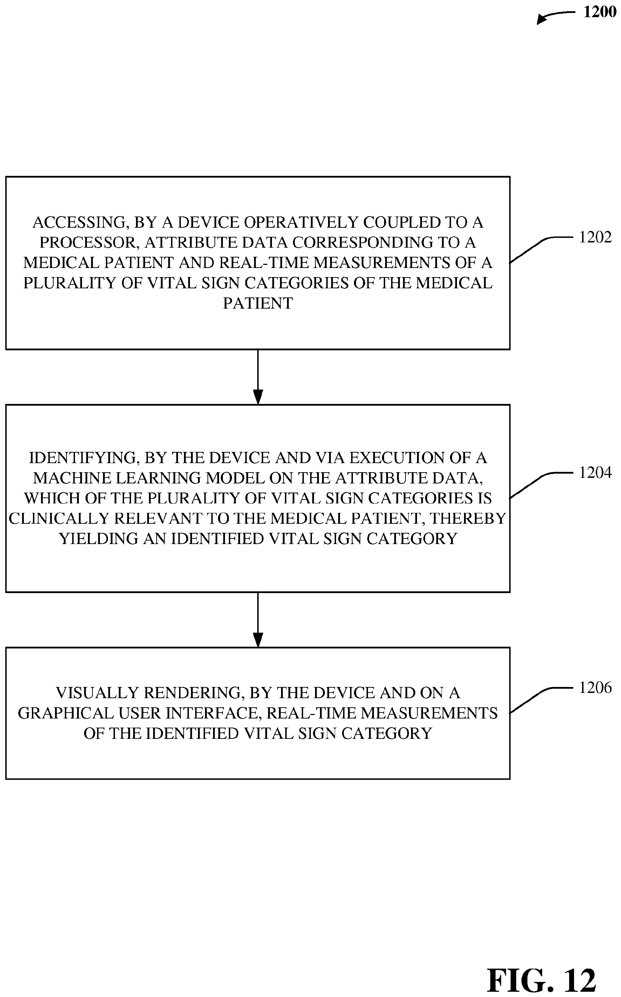

FIG. 12 illustrates a flow diagram of an example, non-limiting computer-implemented method 1200 that can facilitate intelligent clinical user interfaces in accordance with one or more embodiments described herein. In various cases, the intelligent clinical user interface system 102 can facilitate the computer-implemented method 1200.

In various embodiments, act 1202 can include accessing, by a device (e.g., via 114) operatively coupled to a processor (e.g., 110), attribute data (e.g., 104) corresponding to a medical patient and real-time measurements (e.g., 108) of a plurality of vital sign categories (e.g., 106) of the medical patient.

In various aspects, act 1204 can include identifying, by the device (e.g., via 116) and via execution of a machine learning model (e.g., 302) on the attribute data, which of the plurality of vital sign categories is clinically relevant to the medical patient, thereby yielding an identified vital sign category (e.g., indicated by 402).

In various instances, act 1206 can include visually rendering, by the device (e.g., via 118) and on a graphical user interface (e.g., 502), real-time measurements (e.g., 602) of the identified vital sign category.

Although not explicitly shown in FIG. 12, the computer-implemented method 1200 can comprise: determining, by the device (e.g., via 116) and via the execution of the machine learning model, a visual style (e.g., indicated by 404) that is predicted to aid the medical patient's viewing or an attending clinician's viewing of the graphical user interface, wherein the real-time measurements of the identified vital sign category can be visually rendered according to the visual style.

Although not explicitly shown in FIG. 12, the visual style can include: a font size to use or avoid; a color to use or avoid; a geometric pattern to use or avoid; or a screen brightness or contrast to use or avoid.

Although not explicitly shown in FIG. 12, the computer-implemented method 1200 can comprise generating, by the device (e.g., via 118), an electronic alert (e.g., 604), in response to the real-time measurements of the identified vital sign category failing to satisfy a threshold value (e.g., indicated by 406). In various cases, the computer-implemented method 1200 can comprise: computing, by the device (e.g., via 116) and via the execution of the machine learning model, the threshold value.

Although not explicitly shown in FIG. 12, the graphical user interface can be associated with a mobile computing device or with a hospital console.

Although not explicitly shown in FIG. 12, the graphical user interface can incorporate an augmented reality overlay (e.g., a two-dimensional or three-dimensional reconstructed model of an organ of the medical patient) superimposed over an image of the medical patient (e.g., the image can be captured by a smart phone of the medical patient or by hospital camera that is facing the medical patient).

Although various embodiments are described herein with respect to GUIs, this is a mere non-limiting example for ease of explanation and illustration. In various other embodiments, the teachings described herein can be applied or extrapolated to any suitable electronic user interfaces (e.g., are not limited only to graphical user interfaces). As a non-limiting example, various embodiments described herein can be applied or implemented to an audio user interface. In such cases, the patient-tailored GUI 502 can instead be considered as a patient-tailored audio user interface, the real-time vital sign measurements 602 can be audibly conveyed rather than visually conveyed, and the recommended visual style indicator 404 can instead be considered as a recommended audible style indicator (e.g., can specify aurally stylistic elements, such as sound volume, sound pitch, or sound timber, that are predicted to increase or benefit listening comfort or tolerance of the medical patient). As another non-limiting example, various embodiments described herein can be applied or implemented to a tactile user interface (e.g., an electronic Braille interface). In such cases, the patient-tailored GUI 502 can instead be considered as a patient-tailored tactile user interface, the real-time vital sign measurements 602 can be tactily or haptically conveyed rather than visually conveyed, and the recommended visual style indicator 404 can instead be considered as a recommended tactile style indicator (e.g., can specify tactile stylistic elements, such as sensitivity of haptic feedback, that are predicted to increase or benefit tactile comfort or tolerance of the medical patient). In any case, various embodiments described herein can be considered as facilitating automatic and intelligent customization or tailoring of clinical user interfaces based on patient idiosyncrasies.

Indeed, various embodiments can include a computer program product for facilitating intelligent clinical user interfaces. In various aspects, the computer program product can comprise a non-transitory computer-readable memory (e.g., 112) having program instructions embodied therewith. In various instances, the program instructions can be executable by a processor (e.g., 110) to cause the processor to: access attribute data (e.g., 104) corresponding to a medical patient and real-time measurements (e.g., 108) of a plurality of vital sign categories (e.g., 106) of the medical patient; identify, via execution of a machine learning model (e.g., 302) on the attribute data, which of the plurality of vital sign categories is clinically relevant to the medical patient, thereby yielding an identified vital sign category (e.g., indicated by 402); and convey, via an electronic user interface (e.g., 502 as a non-limiting example), real-time measurements (e.g., 602) of the identified vital sign category.

In some aspects, the electronic user interface can be a graphical user interface (e.g., 502), and the program instructions can be further executable to cause the processor to: determine, via the execution of the machine learning model, a visual style (e.g., 404) that is predicted to aid the medical patient's interaction with the graphical user interface, and the real-time measurements of the identified vital sign category can be rendered according to the visual style.

In other aspects, the electronic user interface can be an auditory user interface (e.g., auditory or audible or aural version of 502), and the program instructions can be further executable to cause the processor to: determine, via the execution of the machine learning model, an aural style (e.g., auditory or audible or aural version of 404) that is predicted to aid the medical patient's interaction with the auditory user interface, and the real-time measurements of the identified vital sign category can be rendered according to the aural style.

In yet other aspects, the electronic user interface can be a tactile user interface (e.g., tactile version of 502), and the program instructions can be further executable to cause the processor to: determine, via the execution of the machine learning model, a tactile style (e.g., tactile version of 404) that can be predicted to aid the medical patient's interaction with the tactile user interface, and the real-time measurements of the identified vital sign category can be rendered according to the tactile style.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ artificial intelligence to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z₁, z₂, z₃, z₄, z*ₙ*), to a confidence that the input belongs to a class, as by f(z) = *confidence*(*class*)*.* Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

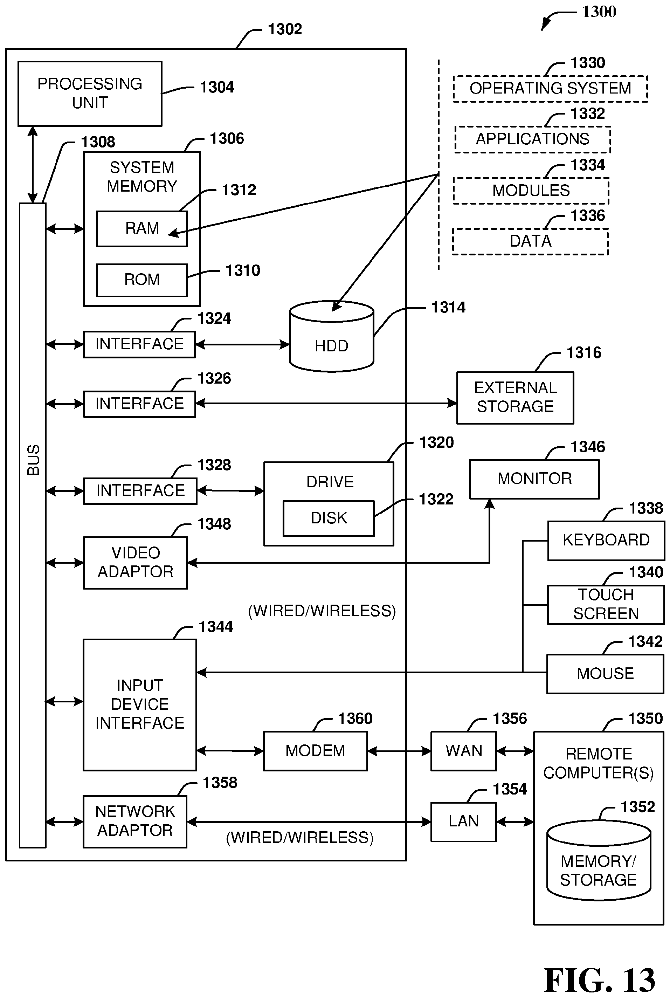

In order to provide additional context for various embodiments described herein, FIG. 13 and the following discussion are intended to provide a brief, general description of a suitable computing environment 1300 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 13, the example environment 1300 for implementing various embodiments of the aspects described herein includes a computer 1302, the computer 1302 including a processing unit 1304, a system memory 1306 and a system bus 1308. The system bus 1308 couples system components including, but not limited to, the system memory 1306 to the processing unit 1304. The processing unit 1304 can be any of various commercially available processors. Dual microprocessors and other multi-processor architectures can also be employed as the processing unit 1304.

The system bus 1308 can be any of several types of bus structure that can further interconnect to a memory bus (with or without a memory controller), a peripheral bus, and a local bus using any of a variety of commercially available bus architectures. The system memory 1306 includes ROM 1310 and RAM 1312. A basic input/output system (BIOS) can be stored in a non-volatile memory such as ROM, erasable programmable read only memory (EPROM), EEPROM, which BIOS contains the basic routines that help to transfer information between elements within the computer 1302, such as during startup. The RAM 1312 can also include a high-speed RAM such as static RAM for caching data.

The computer 1302 further includes an internal hard disk drive (HDD) 1314 (e.g., EIDE, SATA), one or more external storage devices 1316 (e.g., a magnetic floppy disk drive (FDD) 1316, a memory stick or flash drive reader, a memory card reader, etc.) and a drive 1320, e.g., such as a solid state drive, an optical disk drive, which can read or write from a disk 1322, such as a CD-ROM disc, a DVD, a BD, etc. Alternatively, where a solid state drive is involved, disk 1322 would not be included, unless separate. While the internal HDD 1314 is illustrated as located within the computer 1302, the internal HDD 1314 can also be configured for external use in a suitable chassis (not shown). Additionally, while not shown in environment 1300, a solid state drive (SSD) could be used in addition to, or in place of, an HDD 1314. The HDD 1314, external storage device(s) 1316 and drive 1320 can be connected to the system bus 1308 by an HDD interface 1324, an external storage interface 1326 and a drive interface 1328, respectively. The interface 1324 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1302, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1312, including an operating system 1330, one or more application programs 1332, other program modules 1334 and program data 1336. All or portions of the operating system, applications, modules, or data can also be cached in the RAM 1312. The systems and methods described herein can be implemented utilizing various commercially available operating systems or combinations of operating systems.

Computer 1302 can optionally comprise emulation technologies. For example, a hypervisor (not shown) or other intermediary can emulate a hardware environment for operating system 1330, and the emulated hardware can optionally be different from the hardware illustrated in FIG. 13. In such an embodiment, operating system 1330 can comprise one virtual machine (VM) of multiple VMs hosted at computer 1302. Furthermore, operating system 1330 can provide runtime environments, such as the Java runtime environment or the .NET framework, for applications 1332. Runtime environments are consistent execution environments that allow applications 1332 to run on any operating system that includes the runtime environment. Similarly, operating system 1330 can support containers, and applications 1332 can be in the form of containers, which are lightweight, standalone, executable packages of software that include, e.g., code, runtime, system tools, system libraries and settings for an application.

Further, computer 1302 can be enable with a security module, such as a trusted processing module (TPM). For instance with a TPM, boot components hash next in time boot components, and wait for a match of results to secured values, before loading a next boot component. This process can take place at any layer in the code execution stack of computer 1302, e.g., applied at the application execution level or at the operating system (OS) kernel level, thereby enabling security at any level of code execution.

A user can enter commands and information into the computer 1302 through one or more wired/wireless input devices, e.g., a keyboard 1338, a touch screen 1340, and a pointing device, such as a mouse 1342. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1304 through an input device interface 1344 that can be coupled to the system bus 1308, but can be connected by other interfaces, such as a parallel port, an IEEE 1394 serial port, a game port, a USB port, an IR interface, a BLUETOOTH^{®} interface, etc.

A monitor 1346 or other type of display device can be also connected to the system bus 1308 via an interface, such as a video adapter 1348. In addition to the monitor 1346, a computer typically includes other peripheral output devices (not shown), such as speakers, printers, etc.

The computer 1302 can operate in a networked environment using logical connections via wired or wireless communications to one or more remote computers, such as a remote computer(s) 1350. The remote computer(s) 1350 can be a workstation, a server computer, a router, a personal computer, portable computer, microprocessor-based entertainment appliance, a peer device or other common network node, and typically includes many or all of the elements described relative to the computer 1302, although, for purposes of brevity, only a memory/storage device 1352 is illustrated. The logical connections depicted include wired/wireless connectivity to a local area network (LAN) 1354 or larger networks, e.g., a wide area network (WAN) 1356. Such LAN and WAN networking environments are commonplace in offices and companies, and facilitate enterprise-wide computer networks, such as intranets, all of which can connect to a global communications network, e.g., the Internet.

When used in a LAN networking environment, the computer 1302 can be connected to the local network 1354 through a wired or wireless communication network interface or adapter 1358. The adapter 1358 can facilitate wired or wireless communication to the LAN 1354, which can also include a wireless access point (AP) disposed thereon for communicating with the adapter 1358 in a wireless mode.

When used in a WAN networking environment, the computer 1302 can include a modem 1360 or can be connected to a communications server on the WAN 1356 via other means for establishing communications over the WAN 1356, such as by way of the Internet. The modem 1360, which can be internal or external and a wired or wireless device, can be connected to the system bus 1308 via the input device interface 1344. In a networked environment, program modules depicted relative to the computer 1302 or portions thereof, can be stored in the remote memory/storage device 1352. It will be appreciated that the network connections shown are example and other means of establishing a communications link between the computers can be used.

When used in either a LAN or WAN networking environment, the computer 1302 can access cloud storage systems or other network-based storage systems in addition to, or in place of, external storage devices 1316 as described above, such as but not limited to a network virtual machine providing one or more aspects of storage or processing of information. Generally, a connection between the computer 1302 and a cloud storage system can be established over a LAN 1354 or WAN 1356 e.g., by the adapter 1358 or modem 1360, respectively. Upon connecting the computer 1302 to an associated cloud storage system, the external storage interface 1326 can, with the aid of the adapter 1358 or modem 1360, manage storage provided by the cloud storage system as it would other types of external storage. For instance, the external storage interface 1326 can be configured to provide access to cloud storage sources as if those sources were physically connected to the computer 1302.

The computer 1302 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

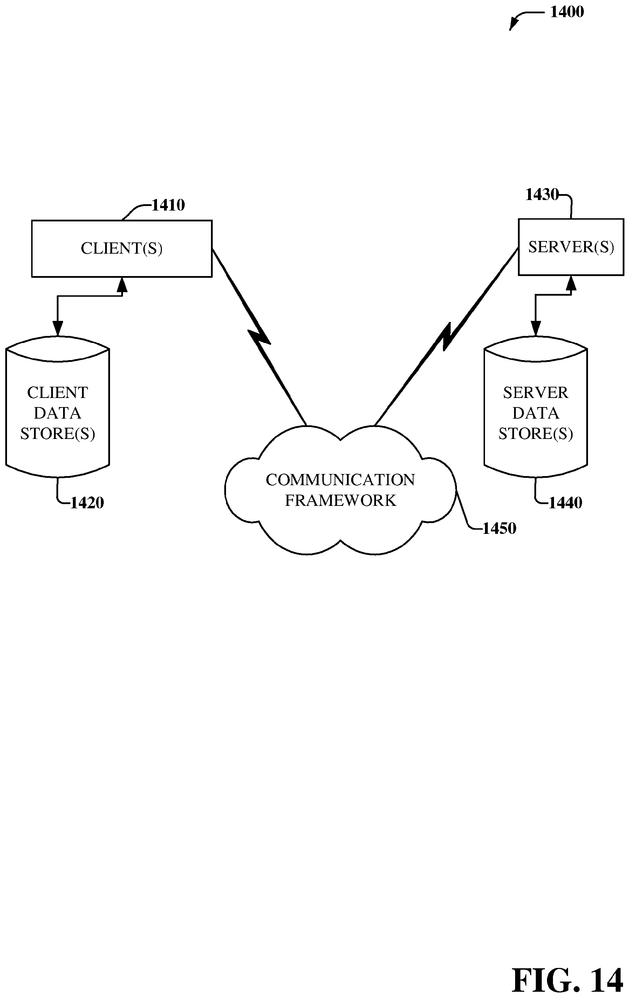

FIG. 14 is a schematic block diagram of a sample computing environment 1400 with which the disclosed subject matter can interact. The sample computing environment 1400 includes one or more client(s) 1410. The client(s) 1410 can be hardware or software (e.g., threads, processes, computing devices). The sample computing environment 1400 also includes one or more server(s) 1430. The server(s) 1430 can also be hardware or software (e.g., threads, processes, computing devices). The servers 1430 can house threads to perform transformations by employing one or more embodiments as described herein, for example. One possible communication between a client 1410 and a server 1430 can be in the form of a data packet adapted to be transmitted between two or more computer processes. The sample computing environment 1400 includes a communication framework 1450 that can be employed to facilitate communications between the client(s) 1410 and the server(s) 1430. The client(s) 1410 are operably connected to one or more client data store(s) 1420 that can be employed to store information local to the client(s) 1410. Similarly, the server(s) 1430 are operably connected to one or more server data store(s) 1440 that can be employed to store information local to the servers 1430.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a standalone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system, comprising:
a processor (e.g., 110) that executes computer-executable components stored in a non-transitory computer-readable memory (e.g., 112), wherein the computer-executable components comprise:
an access component (e.g., 114) that accesses attribute data (e.g., 104) corresponding to a medical patient and that accesses real-time measurements (e.g., 108) of a plurality of vital sign categories (e.g., 106) of the medical patient;
a model component (e.g., 116) that identifies, via execution of a machine learning model (e.g., 302) on the attribute data, which of the plurality of vital sign categories is clinically relevant to the medical patient, thereby yielding an identified vital sign category (e.g., 402); and
a display component (e.g., 118) that visually renders, on a graphical user interface (e.g., 502), real-time measurements (e.g., 602) of the identified vital sign category.

2. The system of claim 1, wherein the model component determines, via the execution of the machine learning model, a visual style (e.g., 404) that is predicted to aid the medical patient's viewing or an attending clinician's viewing of the graphical user interface, and wherein the display component visually renders the real-time measurements of the identified vital sign category according to the visual style.

3. The system of claim 2, wherein the visual style includes: a font size to use or avoid; a color to use or avoid; a geometric pattern to use or avoid; or a screen brightness or contrast to use or avoid.

4. The system of claim 1, wherein the display component generates an electronic alert (e.g., 604), in response to the real-time measurements of the identified vital sign category failing to satisfy a threshold value (e.g., 406).

5. The system of claim 4, wherein the model component computes, via the execution of the machine learning model, the threshold value.

6. The system of claim 1, wherein the graphical user interface is associated with a mobile computing device.

7. The system of claim 1, wherein the graphical user interface is associated with a hospital console.

8. The system of claim 1, wherein the graphical user interface incorporates an augmented reality overlay superimposed over an image of the medical patient.

9. A computer-implemented method, comprising:
accessing, by a device (e.g., via 114) operatively coupled to a processor, attribute data (e.g., 104) corresponding to a medical patient and real-time measurements (e.g., 108) of a plurality of vital sign categories (e.g., 106) of the medical patient;
identifying, by the device (e.g., via 116) and via execution of a machine learning model (e.g., 302) on the attribute data, which of the plurality of vital sign categories is clinically relevant to the medical patient, thereby yielding an identified vital sign category (e.g., 402); and
visually rendering, by the device (e.g., via 118) and on a graphical user interface (e.g., 502), real-time measurements (e.g., 602) of the identified vital sign category.

10. The computer-implemented method of claim 9, further comprising:
determining, by the device (e.g., via 116) and via the execution of the machine learning model, a visual style (e.g., 404) that is predicted to aid the medical patient's viewing or an attending clinician's viewing of the graphical user interface, wherein the real-time measurements of the identified vital sign category are visually rendered according to the visual style.

11. The computer-implemented method of claim 10, wherein the visual style includes: a font size to use or avoid; a color to use or avoid; a geometric pattern to use or avoid; or a screen brightness or contrast to use or avoid.

12. The computer-implemented method of claim 9, further comprising:
generating, by the device (e.g., via 118), an electronic alert (e.g., 604), in response to the real-time measurements of the identified vital sign category failing to satisfy a threshold value (e.g., 406).

13. The computer-implemented method of claim 12, further comprising:
computing, by the device and via the execution of the machine learning model, the threshold value.

14. The computer-implemented method of claim 9, wherein the graphical user interface is associated with a mobile computing device.

15. The computer-implemented method of claim 9, wherein the graphical user interface is associated with a hospital console.
